# EUROPEAN PATENT APPLICATION

(11) **EP 2 374 415 A1**
(43) Date of publication of application: **12.10.2011**
(21) Application number: 11158896.8
(22) Date of filing: 01.09.2004
(51) Int. Cl.: A61B 17/04

(54) **Delivery devices for heart valve repair**

(30) Priority: 04.09.2003 US 656797; 24.11.2003 US 524922 P; 19.12.2003 US 741130; 02.03.2004 US 792681; 27.07.2004 US 901019; 27.07.2004 US 901554; 27.07.2004 US 900980; 27.07.2004 US 901444; 27.07.2004 US 901455; 27.07.2004 US 901555
(62) Divisional of application: 04782847.0
(71) Applicant: Guided Delivery Systems, Inc., Santa Clara CA 95054 (US)
(72) Inventor: Starksen, Niel F, Los Altos Hills, CA 94022 (US); To, John, Newark, CA 94560 (US); Fabro, Mariel, San Jose, CA 95131 (US); Wei, Michael F, Redwood City, CA 94065 (US); Morales, Rodolfo A, Los Gatos, CA 95032 (US)
(74) Representative: Price, Nigel John King

(57) **Abstract**

Devices, systems and methods facilitate positioning of a cardiac valve annulus treatment device, thus enhancing treatment of the annulus. Methods generally involve advancing an anchor delivery device (100) through vasculature of the patient to a location in the heart for treating the valve annulus, contacting the anchor delivery device with a length of the valve annulus, delivering a plurality of coupled anchors from the anchor delivery device to secure the anchors to the annulus, and drawing the anchors together to circumferentially tighten the valve annulus. Devices generally include an elongate catheter having at least one tensioning member and at least one tensioning actuator for deforming a distal portion of the catheter to help it conform to a valve annulus. The catheter device may be used to navigate a subannular space below a mitral valve to facilitate positioning of an anchor delivery device.

## Description

### BACKGROUND OF THE INVENTION

1. Field of the Invention. The present invention relates generally to medical devices and methods. More particularly, the invention relates to devices, systems and methods for enhancing cardiovascular valve repair, especially the repair of heart valves such as the mitral and tricuspid valves.

In recent years, many advances have been made to reduce the invasiveness of cardiac surgery. In an attempt to avoid open, stopped-heart procedures, which may be accompanied by high patient morbidity and mortality, many devices and methods have been developed for operating on a heart through smaller incisions, operating on a beating heart, and even performing cardiac procedures via transvascular access. Different types of cardiac procedures, such as cardiac ablation techniques for treating atrial fibrillation, stenting procedures for atherosclerosis, and valve repair procedures for treating conditions such as mitral valve regurgitation have experienced significant technological advances. In implementing many minimally invasive cardiac surgery techniques, especially beating-heart techniques, one of the most significant challenges is positioning a treatment device (or multiple devices) in a desired location in or around the heart for performing the procedure. Another challenge, once a device is positioned, is to effectively deploy a given treatment into or on the target cardiac tissue.

One type of cardiac surgery which may benefit from less invasive techniques is heart valve repair. Traditional treatment of heart valve stenosis or regurgitation, such as mitral or tricuspid regurgitation, typically involves an open-heart surgical procedure to replace or repair the valve. Valve repair procedures typically involve annuloplasty, a set of techniques designed to restore the valve annulus shape and strengthen the annulus. Conventional annuloplasty surgery generally requires a large incision into the thorax of the patient (a thoracotomy), and sometimes a median sternotomy (cutting through the middle of the sternum). These open heart, open chest procedures routinely involve placing the patient on a cardiopulmonary bypass machine for sustained periods so that the patient's heart and lungs can be artificially stopped during the procedure. Finally, valve repair and replacement procedures are typically technically challenging and require a relatively large incision through the wall of the heart to access the valve.

Due to the highly invasive nature of open heart valve repair or replacement, many patients, such as elderly patients, patients having recently undergone other surgical procedures, patients with comorbid medical conditions, children, late-stage heart failure patients, and the like, are often considered too high-risk to undergo heart valve surgery and are relegated to progressive deterioration and cardiac enlargement. Often, such patients have no feasible alternative treatments for their heart valve conditions.

To obviate this situation, a number of devices and methods for repairing cardiac valves in a less invasive manner have been described. Some devices provide for heart valve repair through minimally invasive incisions or intravascularly, while others improve upon open heart surgical procedures on beating hearts, stopped hearts or both. As mentioned above, difficulties in performing minimally invasive intracardiac surgery include positioning a minimally invasive treatment device in a desired location for performing a procedure and effectively deploying a given treatment into or on the target cardiac tissue. In heart valve repair procedures, for example, it is often essential for a physician to secure one or more treatment devices to valve annulus tissue. Annular tissue tends to be more fibrous than surrounding muscular or valve leaflet tissue, thus providing a more suitable location for securing such treatment devices, such as anchors, to treat a heart valve. Positioning an anchor deliver device in a desired location adjacent the annular tissue may often be challenging, especially in an intravascular procedure when visualization of the location is limited.

Devices and methods that address these difficulties are described in U.S. Patent Application Serial Nos. 10/792,681, 101741,130, 10/656,797, 10/461,043, 60/388,935, 60/429,288, 60/445,890, 60/462,502 and 60/524,622, which were previously incorporated by reference. For example, these references describe devices and methods for exposing, stabilizing and/or performing a procedure on a heart valve annulus, such as a mitral valve annulus. Many of the devices and methods previously described by the inventors have been found to be highly effective, but improvements are still being sought.

Therefore, it would be beneficial to have improved methods, devices and systems for enhancing heart valve annulus treatment procedures. Ideally, such methods, devices and systems would facilitate positioning of one or more devices in a left ventricle or elsewhere for performing a procedure on a heart valve annulus, visualizing the annulus and/or the like. Additionally, such methods, devices and systems would ideally be introduced intravascularly. At least some of these objectives will be met by the present invention.

2. Description of the Background Art. Published U.S. Application Nos. 2002/0156526, 2003/0220685, 2004/0019378, 2004/0003819, 2004/0030382 and 2004/0039442, and U.S. Patent Nos. 6,629,534 and 6,619,291 describe catheter-based methods for performing annuloplasty. Published U.S. Application 2002/0042621 describes a heart valve annuloplasty system with constrictable plication bands which are optionally attached to a linkage strip. Published U.S. Application 2002/0087169 describes a remote controlled catheter system which can be used to deliver anchors and a tether for performing an annuloplasty procedure. Other patent publications of interest include WO01/26586; US2001/0005787; US2001/0014800; US2002/0013621; US2002/0029080; US2002/0035361; US2002/0042621; US2002/0095167; and US2003/0074012. U.S. patents of interest include 4,014,492; 4,042,979; 4,043,504; 4,055,861; 4,700,250; 5,366,479; 5,450,860; 5,571,215; 5,674,279; 5,709,695; 5,752,518; 5,848,969;5,860,992; 5,904,651; 5,961,539; 5,972,004; 6,165,183; 6,197,017; 6,250,308; 6,260,552; 6,283,993; 6,269,819; 6,312,447; 6,332,893; and 6,524,338. Publications of interest include De Simone et al. (1993) Am. J. Cardiol. 73:721-722, and Downing et al. (2001) Heart Surgery Forum, Abstract 7025. All of the above-cited references are hereby incorporated by reference in the present application.

### BRIEF SUMMARY OF THE INVENTION

Devices, systems and methods of the present invention are generally used to facilitate transvascular, minimally invasive and other "less invasive" surgical procedures, by facilitating the delivery of treatment devices at a treatment site. "Less invasive," for the purposes of this application, means any procedure that is less invasive than traditional, large-incision, open surgical procedures. Thus, a less invasive procedure may be an open surgical procedure involving one or more relatively small incisions, a procedure performed via transvascular percutaneous access, a transvascular procedure via cut-down, a laparoscopic or other endoscopic procedure, or the like. Generally, any procedure in which a goal is to minimize or reduce invasiveness to the patient may be considered less invasive. Furthermore, although the terms "less invasive" and "minimally invasive" may sometimes be used interchangeably in this application, neither these nor terms used to describe a particular subset of surgical or other procedures should be interpreted to limit the scope of the invention. Generally, devices and methods of the invention may be used in performing or enhancing any suitable procedure.

The present application typically describes devices, systems and methods for performing heart valve repair procedures, and more specifically heart valve annuloplasty procedures such as mitral valve annuloplasty to treat mitral regurgitation. Devices and methods of the invention, however, may be used in any suitable procedure, both cardiac and non-cardiac. For example, they may be used in procedures to repair any heart valve, to repair an atrial-septal defect, to access and possibly perform a valve repair or other procedure from (or through) the coronary sinus, to place one or more pacemaker leads, to perform a cardiac ablation procedure such as ablating around pulmonary veins to treat atrial fibrillation, and/or the like. In other embodiments, the devices and methods may be used to enhance a laparoscopic or other endoscopic procedure on any part of the body, such as the bladder, stomach, gastroesophageal junction, vasculature, gall bladder, or the like. Therefore, although the following description typically focuses on mitral valve and other heart valve repair, such description should not be interpreted to limit the scope of the invention as defined by the claims.

That being said, the present invention generally provides devices, systems and methods for enhanced treatment of a cardiac valve annulus such as a mitral valve annulus. Methods generally involve contacting an anchor delivery device with a length of a valve annulus, delivering a plurality of coupled anchors from the anchor delivery device to secure the anchors to the annulus, and drawing the anchors together to circumferentially tighten the annulus. One device generally includes an elongate catheter having a housing at or near the distal end for releasably housing a plurality of coupled anchors. The device may be positioned such that the housing abuts or is close to valve annular tissue, such as at an intersection of the left ventricular wall and one or more mitral valve leaflets of the heart. Some embodiments include self-securing anchors, which may change from undeployed to deployed configurations. Anchors may be drawn together to tighten the annulus by cinching a tether slidably coupled with the anchors and/or by a self-deforming member coupled with the anchors. Another device includes a steerable guide catheter for helping position the anchor delivery device for treating a valve annulus.

In many cases, methods of the present invention will be performed on a beating heart. Access to the beating heart may be accomplished by any available technique, including intravascular, transthoracic, and the like. Intravascular access to a heart valve may be achieved using any suitable route or method. To perform a procedure on a mitral valve, for example, in one embodiment a catheter may be advanced through a femoral artery, to the aorta, and into the left ventricle of the heart, to contact a length of the mitral valve. Alternatively, access may be gained through the venous system, to a central vein, into the right atrium of the heart, and across the interatrial septum to the left side of the heart to contact a length of the mitral valve. In either of these two types of intravascular access, the catheter will often easily be advanced, once it enters the left side of the heart, into a space defined by the left ventricular wall, one or more mitral valve leaflets, and chordae tendineae of the left ventricle. This space provides a convenient conduit for further advancement of the catheter to a desired location for performing mitral valve repair. In alternative embodiments, a catheter device may access the coronary sinus and a valve procedure may be performed directly from the sinus. Furthermore, in addition to beating heart access, methods of the present invention may be used for intravascular stopped heart access as well as stopped heart open chest procedures. Any suitable intravascular or other access method is contemplated within the scope of the invention.

According to one aspect of the present invention, a device for applying coupled anchors to an annulus of a heart valve comprises: an elongate shaft having a proximal end and a distal end; a housing adjacent the distal end; a plurality of coupled anchors disposed within the housing; at least one anchor contacting member for causing the anchors to be delivered from the housing; and at least one actuator at or near the proximal end of the shaft for affecting the anchor contacting member to cause delivery of the anchors to the valve annulus.

In some embodiments, the elongate shaft comprises a flexible catheter which is advancable intravascularly to the heart. In a preferred embodiment, a flexible elongate shaft has a diameter of about 5 French (1.67 mm) or less and deploys anchors having a radius, when deployed, of about 3 mm or more. The housing itself may house any suitable number of anchors. In one embodiment, for example, the housing holds between 1 anchor and 20 anchors, and more preferably about 3-10 anchors, and in one embodiment 10 anchors. Also in some embodiments, the housing is sufficiently flexible to allow the housing to conform to the annulus. For example, the housing may conform to the annulus at an intersection of a left ventricular wall and one or more mitral valve leaflets of the heart. The housing may thus be positioned or advanced through the subvalvular space as discussed above. In some embodiments, the housing is coupled with an actuator for deforming the housing to conform it to the annulus. The housing may have any suitable configuration, but in some embodiments it has a cross section with a shape that is roughly semi-circular, circular, oval, part of an oval, a partial or complete ellipse, or the like. For example, a housing with an elliptical shape may sometimes be used to help ensure that an anchor delivering surface of the housing comes into contact with the annular tissue. In various embodiments, the housing may have one or multiple openings for allowing egress of the anchors. In one embodiment, for example, the housing has multiple openings, each opening suitable for egress of one anchor.

In some embodiments, the housing includes a shape-changing portion, typically a distal portion. Such embodiments may further include a first tensioning cord coupled with the shape-changing portion for applying tension to the shape-changing portion to cause it to bend in at least a first direction. Optionally, a second tensioning cord may be coupled with the shape-changing portion for applying tension to the shape-changing portion to cause it to bend in at least a second direction. The first direction, for example, may be approximately a C-shape for conforming to the annulus and the second direction comprises an upward or proximal direction for applying force to the annulus. In some embodiments, the shape-changing portion includes multiple notches along at least one side to control bending into a curve which conforms to the shape of the annulus. Alternatively, the shape-changing portion may comprise multiple stacked segments coupled with at least the first tensioning member to control bending into the shape of the annulus. In other embodiments, the shape-changing portion comprises a shape-memory material configured to conform to the shape of the annulus. In some embodiments, the shape-changing portion further comprises at least one lumen for introducing a fluid to cause the shape-memory material to conform to the shape of the annulus. The distal portion of the housing may alternatively be coupled with a shaped expandable balloon for deforming the distal portion. In some embodiments, the housing may be coupled with an expandable member such that when the expandable member expands, it helps wedge, drive or press the housing against valve annulus tissue. For example, such an expandable member may help to wedge a housing into the corner formed by a ventricular wall and a valve leaflet.

As explained above, anchors of the device may have any suitable shape, size and properties and may be made of any suitable materials. Anchors may be self-deforming in some embodiments, thus having an undeployed shape when constrained in the housing of the delivery device and assuming a deployed shape after release from the housing. In one embodiment, each of the plurality of coupled anchors has a generally straight configuration, with two sharpened tips and a loop between the two. Upon deployment, such an anchor may curve, with each tip curving in an opposite direction to bite into tissue. The loop, in turn, may act as an eye for a tether. In another embodiment, each anchor may have a C-shaped or semicircular undeployed shape and an overlapping circle or key ring deployed shape. In such an embodiment, the open ends of the C are typically sharpened, to enable the anchor to enter tissue of a valve annulus. As the C-shaped anchor contacts and enters the tissue, it also closes, and the ends overlap to form a circle or key-ring-shaped deployed anchor. Such an anchor may be applied such that it rests flush with the surface of the annular tissue without protruding sharp ends or other parts. The anchors may be made of Nitinol, shape-memory stainless steel, or any other super-elastic or shape-memory material. Alternatively, the anchors may be spring loaded or otherwise housed within the housing so as to change from an undeployed to a deployed shape upon release from the housing.

In some embodiments, the anchors are slidably coupled with a tether. In such embodiments, each of the plurality of anchors may include at least one eyelet, with the tether slidably passing through the eyelet of each anchor. Alternatively, the tether may extend along the anchors to be positioned between the anchors and annular tissue upon deployment. In other embodiments, the anchors may be coupled by a self-deforming coupling member fixedly coupled with each anchor. For example, the coupling member (or "backbone") may comprise a Nitinol member having an undeployed shape approximating a straight line and a deployed shape of a line having multiple bends. Upon changing from the undeployed shaped to the deployed shape, the coupling member may cinch the anchors to circumferentially tighten the valve annulus. Some embodiments may include both a tether and a self-deforming coupling member, with both being available to provide cinching of a valve annulus.

In some embodiments, the at least one anchor contacting member comprises at least one retractable force applying device which, when retracted proximally relative to the housing, sequentially contacts the anchors to apply force to the anchors such that they exit the housing via at least one opening in the housing. Such a force applying device, for example, may comprise a ball, plate, anchor, hook, plunger or the like, coupled with a cord, wire, tether or the like. When the tether is pulled proximally, the ball contacts the distal-most anchor in the delivery device and forces it out an opening in the device. When retracted further, the ball then contacts the next anchor, forcing it out, and so on. In alternative embodiments, the at least one anchor contacting member comprises at least one movable retaining member. For example, such a movable retaining member may comprise one or more anchor retaining mandrels, slidably disposed in the housing so that retracting the mandrel(s) releases one or more of the anchors. Sometimes, for example, two mandrels are positioned in the housing to retain two arms of each anchor, for example when the undeployed shape of each anchor is approximately a C-shape or semicircle. The mandrel (or mandrels) may typically be retracted to release anchors one at a time, in groups, or all at once.

In some embodiments, the at least one actuator includes means for cinching the coupled anchors to reduce the circumference of the valve annulus. Such an actuator may comprise, for example, a trigger, a handle, a plunger, a squeeze-activated device, a syringe-grip device, a foot-operated device, or the like. Some embodiments of the device also include at least one expandable member disposed within the housing for pushing the anchors out of the housing.

In yet another aspect of the invention, a device for applying multiple tethered anchors to an annulus of a heart valve comprises: a flexible elongate catheter having a distal portion for delivering the tethered anchors, the distal portion having a cross-sectional diameter of about 1.67 mm or less; a plurality of tethered anchors disposed within the distal portion, each anchor having a radius of at least about 3 mm when deployed from the housing; and at least one anchor delivery member coupled with the catheter for causing the anchors to be delivered from the catheter.

In another aspect of the invention, a self-securing anchor for attaching to annular tissue of a heart valve comprises a super-elastic or shape-memory material having a relatively elongate undeployed shape allowing the anchor to be disposed within a delivery catheter having a cross-sectional diameter of 1.67 mm or less, and assuming a deployed shape with a radius of at least 3 mm upon its release from the delivery device. Generally, such an anchor may have two sharpened tips of the anchor curve in opposite directions when the anchor is released from the delivery device. Optionally, the anchor may include an eyelet disposed between the two sharpened tips.

In still another aspect of the present invention, a self-securing anchor for attaching to annular tissue of a heart valve comprises a shape-memory material having an opened arcuate undeployed shape and assuming a closed shape with overlapping ends after release from constraint. The undeployed and deployed shapes may be any suitable shapes. In one embodiment, for example, the undeployed shape is approximately a C-shape or semicircle having two sharpened ends, and the deployed shape is a closed circle in which the two ends overlap. In some embodiments, the anchor is configured to lie flush with the annular tissue when secured to the tissue. Any super-elastic or shape-memory material may be used to form the anchor, such as Nitinol or any other suitable material.

According to a further aspect of the present invention, an anchor assembly for use within a patient includes: a catheter-type housing, having an open interior, constructed to be passable through a blood vessel and into a heart; a series of tissue-engageable anchors removably housed within the housing, the anchors comprising a distal anchor and a proximal anchor within the open interior of the housing; and a tether serially coupling the anchors to one another. In one embodiment, at least a portion of the housing is flexible. Such an embodiment may optionally further include means for urging the flexible portion of the housing from a first configuration to a second, radially outwardly expanded, curved configuration. In some embodiments, the housing is steerable.

In some embodiments, the distal anchor is fixed to the tether and the proximal anchor is slidably coupled to the tether. Also in some embodiments, the tether may be located substantially external of the catheter-type housing. In one embodiment, the anchors are self-forming and self-securing. In one embodiment, at least one of the anchors comprises a bioactive agent. Alternatively, at least one of the anchors may comprise an electrode. For example, such an electrode may sense at least one of impedance, temperature, and electrical signals. In another embodiment, the electrode comprises an energy-application electrode to supply energy to tissue at least one of ablation and sub-ablation amounts.

In another aspect of the present invention, an anchor assembly for use within a patient includes: a catheter-type housing, having an open interior, constructed to be passable through a blood vessel and into a heart, at least a portion of the housing being flexible; means for urging the flexible portion of the housing from a first configuration to a second, radially outwardly expanded, curved configuration; a series of tissue-engageable anchors removably housed within the open interior of the housing, the anchors comprising a distal anchor fixed to the tether and a proximal anchor slidably coupled to the tether; the anchors being self-forming and self-securing anchors; and a tether serially coupling the anchors to one another.

In another aspect of the invention, an anchor device for use within a patient includes: an elongate housing having a longitudinal axis; a self-forming tissue-engageable anchor carried by and deployable from of the housing; the anchor having a first part and a second part, the second part having a tissue-piercing tip; the anchor placeable in a relatively straight, undeployed state generally parallel to the longitudinal axis when carried by the housing; and the anchor naturally assuming a curved, tissue-engaging deployed state after being deployed from the housing. In one embodiment, the elongate housing has an open interior; the anchor is carried within and deployable from the open interior; and the housing has an opening sized for passage of the anchor therethrough when the anchor is deployed from the housing.

Optionally, at least a portion of the elongate housing may be flexible. In some embodiments, the elongate housing is steerable. In one embodiment, the anchor comprises the first part and at least two of the second parts extending from the first part. In one embodiment, for example, the second parts extend in directions generally opposite one another when in the deployed state. In one such embodiment, the second parts of said anchor have generally circular or semicircular shapes when in the deployed state.

In some embodiments, the anchor is oriented generally perpendicular to the longitudinal axis when in the deployed state. Also in some embodiments, the second part of the anchor may have a generally circular or semicircular shape when in the deployed state. In some embodiments, the device may further comprise a series of the anchors, the anchors comprising a distal anchor and a proximal anchor, and a tether serially coupling the anchors to one another. In some embodiments, the housing has the diametrical dimension d and the anchor in the deployed state has a diametrical dimension D, and the ratio of D to d is at least about 3.5. In other embodiments, the ratio of D to d is at least about 4.4, and more preferably at least about 7, and even more preferably at least about 8.8. As mentioned above, in some embodiments, the anchor may comprise a bioactive agent or electrode.

In another aspect of the present invention, a tissue anchor assembly comprises: an anchor comprising: a base and a resilient, self-forming, tissue-engaging portion extending from the base, the tissue-engaging portion comprising a tissue-piercing tip; the tissue-engaging portion movable between a curved, unrestrained state and a restrained state; a coupling element coupled to the anchor; a housing; and the anchor releasably mounted to the housing with the tissue-engaging portion in the restrained state, the tissue-engaging portion transferable from the restrained state to the unrestrained state when the anchor is released from the housing so that the tissue-piercing tip passing into tissue secures the anchor to the tissue as the tissue-engaging portion moves to the curved, unrestrained state without any further force applied to the anchor.

In some embodiments, the tissue-engaging portion has a generally circular or semicircular shape when in the unrestrained state. In some embodiments, the tissue-engaging portion is straightened when in the restrained state. Optionally, the anchor may comprise first and second of the tissue-engaging portions. For example, in one embodiment, the first tissue-engaging portion comprises a hook. In another embodiment, each of the first and second tissue-engaging portions comprises a hook. In such an embodiment, for example, the hooks may extend in directions generally opposite one another when in the unrestrained state. Other tissue-engaging portions may also extend in directions generally opposite one another when in the unrestrained state.

In some embodiments, the coupling element comprises a tether coupled to the base of the anchor. In one embodiment, the tether is slidably coupled to the base of the anchor. In another embodiment, the coupling element comprises a flexible string-like element. In such embodiments, the housing may comprise a tubular housing. Optionally, the anchor may be releasably mounted within the tubular housing. Optionally, the device may further include a plurality of anchors with the coupling element coupled to each anchor. In some embodiments, at least one anchor is slidably coupled to the coupling element and another of the anchors is fastened to the coupling element.

In another aspect of the present invention, a tissue anchor assembly comprises: a plurality of anchors, each anchor comprising a base and first and second self-forming, tissue-engaging portions extending from the base, the tissue-engaging portions each comprising a tissue-piercing tip; the tissue-engaging portions each movable between a curved, unrestrained state and a straightened, restrained state; the tissue-engaging portions each having a generally circular or semicircular shape when in the unrestrained state; a tether coupled to the bases of the anchors; at least one said anchor being slidably coupled to the tether and another of said anchors being fastened to the tether; a housing; and the anchors and tether being releasably mounted to the housing with the tissue-engaging portions in the restrained state, the tissue-engaging portions transferable from the restrained state to the unrestrained state when the anchors and tether are released from the housing so that the tissue-piercing tips passing into tissue secures the anchor to the tissue as the tissue-engaging portion moves to the curved, unrestrained state without any further force applied to the anchor.

In another aspect of the invention, a tissue anchor assembly includes: an anchor comprising a base and a resilient, self-forming, tissue-engaging portion extending from the base, the tissue-engaging portion comprising a tissue-piercing tip; the tissue-engaging portion movable between a curved, unrestrained state and a restrained state; a coupling element coupled to the anchor; a housing; the anchor releasably mounted to the housing with the tissue-engaging portion in the restrained state, the tissue-engaging portion transformable from the restrained state to the unrestrained state when the anchor is released from the housing; and the tissue-engaging portion comprising means for pulling the anchor into the tissue as the tissue-piercing tip passes into tissue and the tissue-engaging portion moves to the curved, unrestrained state. In various embodiments, the assembly may include any of the features described above.

In some embodiments, the assembly may further include a plurality of anchors with the coupling element coupled to each anchor. In some embodiments, at least one anchor is slidably coupled to the coupling element and another of said anchors is fastened to the coupling element. In some embodiments, the anchor pulling means comprises means for storing potential energy when in the restrained state. Again, in various embodiments the anchor(s) may comprise a bioactive agent or an electrode. For example, the electrode may sense at least one of impendance, temperature, and electrical signals. Alternatively, the electrode may comprise an energy-application electrode to supply energy to tissue at least one of ablation and sub-ablation amounts.

In another aspect of the present invention, a tissue anchor assembly includes: a plurality of anchors, each anchor comprising a base and a first and second resilient, self-forming, tissue-engaging portions extending from the base, the tissue-engaging portions each comprising a tissue-piercing tip; the tissue-engaging portions each movable between a curved, unrestrained state and a restrained state; the tissue-engaging portions each having a generally circular or semicircular shape when in the unrestrained state; a coupling element coupled to each of the anchors; a tubular housing; the anchors releasably mounted within the housing with the tissue-engaging portions in the restrained state, the tissue-engaging portions transformable from the restrained state to the unrestrained state when the anchors are released from the housing; the tissue-engaging portions comprising means for pulling the anchors into the tissue as the tissue-piercing tips pass into tissue and the tissue-engaging portions move to the curved, unrestrained states; and the anchor pulling means comprising means for storing potential energy when in the restrained state.

According to another aspect of the present invention, an anchor device for use within a patient includes: an elongate carrier constructed to be passable through a blood vessel and into a heart; a series of tissue-engageable anchors releasably carried by the elongate carrier, the anchors comprising a distal anchor, a first intermediate anchor and a proximal anchor; a tether serially coupling the anchors to one another, the tether having a distal end secured to the distal anchor or engageable with the distal anchor when the tether is pulled in a proximal direction; at least the proximal anchor being slidably coupled to the tether; and a separator positioned along the tether between adjacent ones of the anchors to restrict how close the adjacent ones of the anchors can come to one another when the tether is placed in tension.

In some embodiments, the elongate carrier comprises a catheter-type housing having an open interior. For example, the anchors may in some embodiments be releasably housed within the open interior of the housing. In some embodiments, the adjacent anchors are the distal and first intermediate anchors. In some embodiments, the anchors are self-forming and self-securing. Also in some embodiments, the separator comprises a generally tubular member. In such embodiments, the tether may optionally pass through the generally tubular member.

In some embodiments, the separator has a textured outer surface. Also in some embodiments, the separator is nonporous. Alternatively, the separator may be porous. In one embodiment, the porous separator comprises pores, and the pores are provided by cutouts formed in the separator. In an alternative embodiment, the separator may be woven or braided. Such a separator may be rigid in one embodiment. In some embodiments, the separator is effectively axially incompressible. In alternative embodiments, the separator is axially compressible from a first length to a second, compressed length. In some embodiments, the axially compressible separators are joined to one another. In other embodiments, the separator comprises first and second portions engaging the tether and a third portion connecting the first and second portions. For example, the third portion may extend to one side of and generally parallel to the tether.

In one embodiment, the elongate carrier has a longitudinal axis; the anchors are self-forming tissue-engageable anchors; the anchors each have a first part and a second part, the second part having a tissue-piercing tip; the elongate carrier having openings sized for passage of the anchors therethrough; the anchors placeable in a relatively straight, undeployed state generally parallel to the longitudinal axis within the elongate carrier; and the anchors naturally assuming a curved, tissue-engaging deployed state after passing through the opening in the elongate carrier. In some of such embodiments, at least a portion of the elongate carrier is flexible. Also in some embodiments, the elongate carrier is steerable. In some embodiments, at least one of the anchors comprises the first part, and at least two of the second parts extend from the first part. In such embodiments, the second parts may extend in directions generally opposite one another when in the deployed state. The second parts, in turn, may have generally circular or semicircular shapes when in the deployed state.

In some embodiments, the anchors are oriented generally perpendicular to the longitudinal axis when in the deployed state. In some embodiments, each anchor has a generally circular or semicircular shape when in the deployed state. In one embodiment, the elongate carrier has a diametrical dimension d and the anchors in the deployed state have a diametrical dimension D, and the ratio of D to d is at least about 3.5. In other embodiments, the ratio of D to d is at least about 4.4, and more preferably at least about 7, and even more preferably at least about 8.8. In some embodiments, the elongate carrier comprises proximal and distal portions, the anchors being carried by the distal portion, the distal portion being releasable from the proximal portions, the distal portion being pierceable by the anchors.

In another aspect of the present invention, an anchor device for use within a patient comprises: an elongate carrier, having a longitudinal axis, constructed to be passable through a blood vessel and into a heart, at least a portion of the elongate carrier being flexible; a series of self-forming, tissue-engageable anchors releasably carried by the elongate carrier, the anchors comprising a distal anchor, a first intermediate anchor and a proximal anchor; the anchors each having a first part and at least two second parts extending from the first part, each second part having a tissue-piercing tip; the elongate carrier having openings sized for passage of the anchors therethrough; the anchors placeable in a relatively straight, undeployed state generally parallel to the longitudinal axis within the elongate carrier; the anchors naturally assuming a curved, tissue-engaging deployed state after passing through the opening in the elongate carrier; the second parts having generally circular or semicircular shapes when in the deployed state; the anchors being oriented generally perpendicular to the longitudinal axis when in the deployed state; the elongate carrier having a diametrical dimension d and the anchors in the deployed state having a diametrical dimension D, the ratio of D to d being at least 3.5; a tether serially coupling the anchors to one another, the tether having a distal end secured to the distal anchor or engageable with the distal anchor when the tether is pulled in a proximal direction; at least the proximal anchor being slidably coupled to the tether; and a separator positioned along the tether between adjacent ones of the anchors to restrict how close the adjacent ones of the anchors can come to one another when the tether is placed in tension.

According to another aspect of the present invention, a device for facilitating placement of one or more devices in contact with a heart valve annulus comprises: an elongate catheter body having a proximal portion and a distal portion; at least one tensioning member coupled with the proximal portion of the catheter body and extending to the distal portion; and at least one tensioning actuator coupled with the proximal portion and the tensioning member for applying tension to the tensioning member to deform the distal portion to allow it to conform generally to a shape of the valve annulus. Typically, the catheter body may be advanced intravascularly to the heart to contact the annulus. In some embodiments, for example, the catheter body may be advanced through an aorta and into a left ventricle of the heart to contact the valve annulus.

In some embodiments, the proximal portion of the catheter body is relatively stiff compared to the distal portion. Also in some embodiments, the catheter body further comprises a rounded, atraumatic distal tip. The catheter body may optionally further include at least one radiopaque portion at or near the distal tip for enhancing visualization. The catheter body may also include at least one lumen extending through the proximal and distal portions for passing one or more fluids.

In some embodiments, the at least one tensioning member comprises two tensioning members, allowing the distal portion to be deformed in at least two different directions. The at least one tensioning member may be made of an suitable material, such as but not limited to Nitinol, polyester, nylon, polypropylene and/or other polymers. The at least one tensioning actuator, in some embodiments, comprises a knob coupled with the tensioning member, wherein turning the knob in one direction applies tension to the tensioning member to deform the distal portion, and wherein turning the knob in an opposite direction releases tension from the tensioning member to return to the distal portion to a less deformed configuration.

Some embodiments of the device further include at least one urging member coupled with the distal portion of the catheter body for urging the distal portion into contact with the valve annulus. For example, the at least one urging member may comprise an expandable member for expanding within a space in a left ventricle formed by a left ventricular wall, at least one mitral valve leaflet and chordae tendiniae of the heart. In an alternative embodiment, the at least one urging member comprises at least one magnet coupled with the distal portion for applying attractive magnetic force between itself and an oppositely charged magnet disposed in a coronary sinus adjacent the valve annulus.

Some embodiments further include a housing coupled with the proximal end of the catheter body, wherein the tensioning actuator is coupled with the housing. Optionally, the housing may further comprise at least one fluid inlet port in fluid communication with at least one lumen in the elongate shaft for introducing one or more fluids into the lumen(s).

In another aspect of the present invention, a system for facilitating placement of one or more devices in contact with a heart valve annulus includes: a shaped guide catheter having at least one curve toward a distal end for positioning the distal end in a position below the mitral valve; a steerable guide catheter passable through the shaped guide catheter and having a steerable distal end for advancing around a length of the valve annulus below the mitral valve; and a guide sheath passable over the steerable guide catheter through the shaped guide catheter, wherein the one or more devices are passable through the guide sheath to contact the mitral valve annulus. Generally, the shaped guide catheter, steerable guide catheter and guide sheath may have any of the various functions and features described above, in various embodiments.

In one embodiment, for example, the shaped guide catheter includes a proximal curve approximately perpendicular to a central axis of the shaped guide catheter for bringing the distal end of the catheter into a plane approximately parallel with a plane of the mitral valve and a distal curve having a radius of curvature approximately the same as a radius of curvature of the mitral valve annulus. In one embodiment, the steerable guide catheter comprises: an elongate catheter body having a proximal portion and a distal portion; at least one tensioning member coupled with the proximal portion of the catheter body and extending to the distal portion; and at least one tensioning actuator coupled with the proximal portion and the tensioning member for applying tension to the tensioning member to deform the distal portion to allow it to conform generally to a shape of the valve annulus. In various embodiment, this steerable guide catheter may have any of the features of the catheter device described above.

In some embodiments of the system, a distal portion of the guide sheath is detachable from a proximal portion of the guide sheath to remain in attached to the valve annulus after an annulus treatment procedure. For example, the detachable distal portion may comprise a tubular member comprising Dacron or the like. In some embodiments, the detachable distal portion is cinchable to tighten the mitral valve annulus.

In some embodiments, the system may further include at least one urging member coupled with at least one of the shaped guide catheter, the steerable guide catheter and the guide sheath. For example, the urging member may comprise an expandable member for expanding within a space in a left ventricle formed by a left ventricular wall, at least one mitral valve leaflet and chordae tendiniae of the heart. Alternatively, the urging member may comprise at least one magnet coupled with at least one of the shaped guide catheter, the steerable guide catheter and the guide sheath for applying attractive magnetic force between itself and an oppositely charged magnet disposed in a coronary sinus adjacent the valve annulus.

Any suitable device or combination of devices may be advanced into contact with the mitral valve annulus in various embodiments. In some embodiments, for example, the system includes an anchor delivery device passable through the guide sheath to contact and apply coupled anchors to the mitral valve annulus. The system may additionally or alternatively include a visualization device passable through the guide sheath to facilitate visualization of the mitral valve annulus. For example, the visualization device may comprise, but is not limited to, an ultrasound device, a camera, an endoscope or a fiber optic device.

According to a further aspect of the present invention, a device for performing a procedure on heart valve annulus includes: a flexible, elongate catheter having a proximal portion and a shaped distal portion, the distal portion having at least one aperture for allowing passage of tissue anchors; multiple tissue anchors releasably housed in the shaped distal portion; at least one cinchable tether slidably coupled with the tissue anchors; and at least one anchor delivery member housed in the distal portion for delivering the anchors out of the at least one aperture to engage tissue of the valve annulus.

In some embodiments, the shaped distal portion comprises at least a first curve. The first curve may have any suitable shape, radius of curvature or the like, such as in one embodiment where the first curve has a radius of curvature between 1.27 cm and 3.81 cm. In some embodiments, the shaped distal portion further comprises a second curve. The first and second curves may be adapted to orient a distal-most portion of the catheter at an angle of between 45° and 90°, relative to the proximal portion of the catheter immediately adjacent the distal portion. In some embodiments, the second curve has a radius of curvature approximating a radius of curvature of the heart valve. In an alternative embodiment, the second curve has a radius of curvature greater than a radius of curvature of the heart valve, wherein the greater radius of curvature causes the distal portion to press outward against the valve annulus. Optionally, at least one of the first and second curves may have the same shape as a corresponding curve in a guide catheter used for delivering the elongate catheter into contact with the valve annulus. For example, first and second curves in the elongate catheter may correspond to first and second corresponding curves in the guide catheter. In such embodiments, the elongate catheter, when advanced through the guide catheter, is oriented such that the at least one aperture contacts the valve annulus tissue.

Some embodiments of the device further include at least one stabilizing member coupled with the elongate catheter for maintaining the distal portion in contact with valve annulus tissue. In some embodiments, for example, the stabilizing member comprises a spiral-shaped member extending from the distal portion to press against heart wall tissue, thus urging the distal portion against the annulus tissue. Optionally, a distal end of the spiral-shaped member may be adapted to engage and press against a junction of one or more papillary muscles and heart wall tissue. In other embodiments, the stabilizing member comprises an arch-shaped shape-memory or spring-loaded member extending from the distal portion to press against heart wall tissue, thus urging the distal portion against the annulus tissue. In one embodiment, a portion of the arch-shaped member is adapted to engage and press against a junction of one or more papillary muscles and heart wall tissue. The device may optionally further include an expandable member coupled with the arch-shaped member for inflating to further press against the heart wall tissue. In other embodiment, the stabilizing member comprises multiple springs extending from the distal portion to press against heart wall tissue, thus urging the distal portion against the annulus tissue. Alternatively, the stabilizing member may include a curved balloon coupled with the distal portion of the catheter, the curved balloon having a greater radius of curvature than a radius of curvature of the valve annulus. Inflating the balloon thus urges the distal portion against the annulus tissue.

In some embodiments, the device further includes at least one termination member for enhancing attachment of a terminal tissue anchor to the heart valve annulus. In some embodiment, the termination member is slidably couplable with the tether. In some embodiments, the termination member is coupled with the tether via a termination catheter device. The termination member may include, for example, at least one deployable tissue attachment member deployable from a retracted configuration for delivery to an expanded configuration for attachment to the valve annulus tissue. The tissue attachment members may include a plurality of members, such as but not limited to barbs, points, needles, hooks, tines, rakes, wires, teeth and/or the like. In some embodiments, the tissue attachment members comprise a shape-memory or super-elastic material. The tissue attachment members may, for example, be disposed circumferentially about a cylindrical member adapted to slide over the tether. Alternatively, the tissue attachment members may be disposed along a portion of a cylindrical member adapted to slide over the tether.

In some embodiments, the tissue attachment members further include a pusher for pushing the tissue attachment member out of the elongate catheter and into the valve annulus tissue. In some embodiments, the tissue attachment members are adapted to engage the terminal tissue anchor. In one embodiment, the tissue attachment members are adapted to enter into the valve annulus tissue in a direction from the terminal tissue anchor toward the other tissue anchors. Optionally, the tissue attachment members may further include a fiber, matrix, textile or mesh disposed on at least a portion of the tissue attachment member for enhancing tissue in-growth over the tissue attachment member.

In some embodiments, each of the tissue anchors comprises at least one tissue engagement feature for preventing the anchors from being pulled out of the valve annulus tissue when the tether is cinched. For example, the tissue engagement feature(s) may include a barb on each end of each tissue anchor. Such a barb may face inward toward a center of the anchor or outward away from the center. In another embodiment, the tissue engagement features comprise a plurality of bends in each anchor. Optionally, each of the tissue anchors may include at least one support member for preventing the anchors from being pulled out of the valve annulus tissue when the tether is cinched. For example, the support member may comprise an attachment point of one arm of each anchor to another arm of each anchor, the attachment point positioned adjacent an eyelet of the anchor. Optionally, a constraining member may be disposed over the attachment point to provide further support. The constraining member may include, for example, a band, tie, sleeve, belt or the like. In some embodiments of the device, each of the tissue anchors comprises at least one tissue adhesion feature adapted to enhance attachment of the tissue anchors to the valve annulus tissue. For example, the tissue adhesion feature may comprise one or more materials disposed over at least part of each anchor, the material(s) selected to promote encapsulation of the anchors within the valve annulus tissue. In one embodiment, the anchor delivery member comprises an anchor contacting member for contacting and urging the anchors out of the at least one aperture and a pull cord coupled with the anchor contacting member for applying force to the anchor contacting member to contact and urge the anchors.

In another aspect of the invention, a device for constricting a valve annulus in a heart includes a plurality of slidably coupled tissue anchors, each anchor including at least one tissue attachment feature for enhancing attachment of the anchor to valve annulus tissue, and at least one cinchable tether slidably coupled with at least some of the tissue anchors and fixedly attached to at least a first of the anchors. The tissue attachment features may comprise any of a number of suitable features. In one embodiment, for example, a surface feature is included along at least a portion of each anchor, such as a porous, textured or coated surface. In other embodiments, the attachment feature comprises at least one circular or hooked portion of each anchor having a small radius of curvature. Alternatively or additionally, the attachment feature may comprise a barb at each end of each anchor.

According to another aspect of the present invention, a catheter assembly includes: a guide sheath having a guide sheath axis; an elongate device slidably extendable within the guide sheath, the elongate device having an elongate device axis; the guide sheath comprising a sheath orientation portion placeable in a chosen shape; and the elongate device comprising a device orientation portion placeable in said chosen shape. In this aspect of the invention, the chosen shape of the sheath and device orientation portions tend to cause the sheath and device orientation portions to assume complementary rotary orientations when the sheath and device orientation portions are axially aligned.

In some embodiments, at least a chosen one of the guide sheath and elongate device are steerable. In some embodiments, the sheath orientation portion is located at a distal end of the guide sheath. Also in some embodiments, the device orientation portion is located at a position spaced apart from a distal end of the elongate device. In some embodiments, the chosen shape comprises a first curved portion and a second curved portion. In some embodiments, the chosen shape comprises a first curved portion in a first plane and a second curved portion in a second plane. Alternatively, said chosen shape may comprise a first curved portion in a first plane and a second curved portion in a second plane, said first and second planes being different planes. In some embodiments, the sheath orientation portion assumes the chosen shape when in a relaxed state. In some embodiments, the sheath and device orientation portions each assume the chosen shape when in a relaxed state.

In another aspect of the present invention, a method for creating a catheter assembly having orientation-seeking inner and outer members comprises: selecting a first chosen location for a sheath orientation portion of a sheath; selecting a second chosen location for an elongate device orientation portion of an elongate device, the elongate device slidably extendable within the guide sheath; creating the sheath orientation portion at the first chosen location of the sheath, the sheath orientation portion placeable in a chosen shape; and creating the elongate device orientation portion at the second chosen location of the sheath, the device orientation portion placeable in said chosen shape, whereby the chosen shape of the sheath and device orientation portions tend to cause the sheath and device orientation portions to assume complementary rotary orientations when the sheath and device orientation portions are axially aligned.

In some embodiments, the first chosen location selecting step comprises selecting the distal end of the guide sheath as the first chosen location. Also in some embodiments, the second chosen location selecting step comprises selecting a position spaced apart from a distal end of the elongate device as the second chosen location. In other embodiments, the sheath orientation portion creating step comprises forming a first curved portion and a second curved portion. Optionally, the sheath orientation portion creating step may involve forming a first curved portion in a first plane and a second curved portion in a second plane. In some embodiments, the forming step is carried out with said first and second planes being different planes. In some embodiments, the sheath orientation portion creating step is carried out with the sheath orientation portion naturally assuming the chosen shape when in a relaxed state.

In another aspect of the present invention, a method for orienting the inner and outer members of a catheter assembly involves: selecting a catheter assembly comprising: a guide sheath having a guide sheath axis and a distal end; an elongate device slidably extendable within the guide sheath, the elongate device having an elongate device axis; the guide sheath comprising a sheath orientation portion, the sheath orientation portion placeable in a chosen shape; and the elongate device comprising a device orientation portion, the device orientation portion placeable in said chosen shape; placing the distal end of the guide sheath at a chosen location; axially aligning the sheath and device orientation portions so that the guide sheath and elongate device are at a chosen axial orientation; and axially repositioning, as necessary, the guide sheath and elongate device away from and back into the chosen axial orientation until the sheath and device orientation portions to assume complementary rotary orientations. In some embodiments, the catheter assembly selecting step is carried out with the first chosen location at the distal end of the guide sheath. In some embodiments, the axially aligning step is carried out with the distal end of the elongate device at a position distal of the distal end of the guide sheath.

In another aspect of the present invention, a method for creating a catheter assembly having orientation-seeking inner and outer members comprises: selecting a first chosen location for a sheath orientation portion of a sheath; selecting a second chosen location for an elongate device orientation portion of an elongate device, the elongate device slidably extendable within the guide sheath; creating the sheath orientation portion at the first chosen location of the sheath, the sheath orientation portion placeable in a chosen shape; and creating the elongate device orientation portion at the second chosen location of the sheath, the device orientation portion placeable in said chosen shape, whereby the chosen shape of the sheath and device orientation portions tend to cause the sheath and device orientation portions to assume complementary rotary orientations when the sheath and device orientation portions are axially aligned.

In some embodiments, the first chosen location selecting step comprises selecting the distal end of the guide sheath as the first chosen location. Also in some embodiments, the second chosen location selecting step comprises selecting a position spaced apart from a distal end of the elongate device as the second chosen location. In other embodiments, the sheath orientation portion creating step comprises forming a first curved portion and a second curved portion. Optionally, the sheath orientation portion creating step may involve forming a first curved portion in a first plane and a second curved portion in a second plane. In some embodiments, the forming step is carried out with said first and second planes being different planes. In some embodiments, the sheath orientation portion creating step is carried out with the sheath orientation portion naturally assuming the chosen shape when in a relaxed state.

In another aspect of the present invention, a method for orienting the inner and outer members of a catheter assembly involves: selecting a catheter assembly comprising: a guide sheath having a guide sheath axis and a distal end; an elongate device slidably extendable within the guide sheath, the elongate device having an elongate device axis; the guide sheath comprising a sheath orientation portion, the sheath orientation portion placeable in a chosen shape; and the elongate device comprising a device orientation portion, the device orientation portion placeable in said chosen shape; placing the distal end of the guide sheath at a chosen location; axially aligning the sheath and device orientation portions so that the guide sheath and elongate device are at a chosen axial orientation; and axially repositioning, as necessary, the guide sheath and elongate device away from and back into the chosen axial orientation until the sheath and device orientation portions to assume complementary rotary orientations. In some embodiments, the catheter assembly selecting step is carried out with the first chosen location at the distal end of the guide sheath. In some embodiments, the axially aligning step is carried out with the distal end of the elongate device at a position distal of the distal end of the guide sheath.

These and other aspects and embodiments are described more fully below with reference to the drawing figures.

### BRIEF DESCRIPTION OF THE DRAWINGS

Fig. 1 is a cross-sectional view of a heart with a flexible anchor delivery device being positioned for treatment of a mitral valve annulus, according to one embodiment of the present invention;

Figs. 2A and 2B are cross-sectional views of a portion of a heart, schematically showing positioning of a flexible device for treatment of a mitral valve annulus, according to one embodiment of the present invention;

Figs. 2C and 2D are cross-sectional views of a portion of a heart, showing positioning of a flexible anchor delivery device for treatment of a mitral valve annulus, according to one embodiment of the present invention;

Fig. 3 is a perspective view of a distal portion of an anchor delivery device, according to one embodiment of the invention;

Fig. 4 is a perspective view of a segment of a distal portion of an anchor delivery device, with anchors in an undeployed shape and position;

Fig. 5 is a different perspective view of the segment of the device shown in Fig. 4;

Fig. 6 is a perspective view of a segment of a distal portion of an anchor delivery device, with anchors in a deployed shape and position;

Figs. 7A-7E are cross-sectional views of an anchor delivery device, illustrating a method for delivering anchors to valve annulus tissue, according to one embodiment of the invention;

Figs. 8A and 8B are top-views of a plurality of anchors coupled to a self-deforming coupling member or "backbone," with the backbone shown in an undeployed shape and a deployed shape;

Figs. 9A-9C are various perspective views of a distal portion of a flexible anchor delivery device according to one embodiment of the present invention;

Figs. 10A-10F demonstrate a method for applying anchors to a valve annulus and cinching the anchors to tighten the annulus, using an anchor delivery device according to an embodiment of the invention;

Fig. 11 shows a heart in cross-section with a guide catheter device advanced through the aorta into the left ventricle according to an embodiment of the invention;

Fig. 11A shows a distal end of an anchor delivery device passing through a guide catheter according to an embodiment of the invention;

Fig. 11B shows middle portions of an anchor delivery device and a guide catheter having corresponding orientation portions according to an embodiment of the invention;

Figs. 12A-12D show various embodiments of support members for supporting an anchor delivery device against a valve annulus;

Figs. 13A-13C show a device and method for facilitating termination and load distribution of a series of anchors according to one embodiment of the invention;

Figs. 14A-14F demonstrate a method for advancing an anchor delivery device to a position for treating a heart valve according to an embodiment of the invention;

Figs. 15A and 15B are side cross-sectional views of a guide catheter device for facilitating positioning of an anchor delivery device according to an embodiment of the invention;

Figs. 16A-16E show improved tissue anchors according to various embodiments of the present invention;

Figs. 17A-17C show a self-forming anchor attaching to tissue of a valve annulus according to one embodiment of the present invention;

Figs. 18 shows a self-forming anchor attaching to tissue of a valve annulus according to another embodiment of the present invention;

Fig. 19A shows an anchor device having a sleeve between two adjacent anchors according to one embodiment of the invention; and

Fig. 19B shows an anchor device having a sleeve between three anchors according to one embodiment of the invention.

### DETAILED DESCRIPTION OF THE INVENTION

Devices, systems and methods of the present invention are generally used to facilitate transvascular, minimally invasive and other "less invasive" surgical procedures, by facilitating the delivery of treatment devices at a treatment site. Although the following description focuses on use of devices and methods of the invention for mitral valve repair, the devices and methods may be used in any suitable procedure, both cardiac and non-cardiac. When used for treatment of a cardiac valve annulus, the inventive methods generally involve contacting an anchor delivery device with a length of the valve annulus, delivering a plurality of coupled anchors from the anchor delivery device, and drawing the anchors together to tighten the annulus. Devices include an elongate catheter having a housing at or near the distal end for releasably housing a plurality of coupled anchors, as well as delivery devices for facilitating advancement and/or positioning of an anchor delivery device. Devices may be positioned such that the housing abuts or is close to valve annular tissue, such as in a location within the left ventricle defined by the left ventricular wall, a mitral valve leaflet and chordae tendineae. Self-securing anchors having any of a number of different configurations may be used in some embodiments. Additional devices include delivery devices for facilitating delivery and/or placement of an anchor delivery device at a treatment site.

In many cases, methods of the present invention will be performed on a beating heart. Access to the beating heart may be accomplished by any available technique, including intravascular, transthoracic, and the like. In addition to beating heart access, the methods of the present invention may be used for intravascular stopped heart access as well as stopped heart open chest procedures.

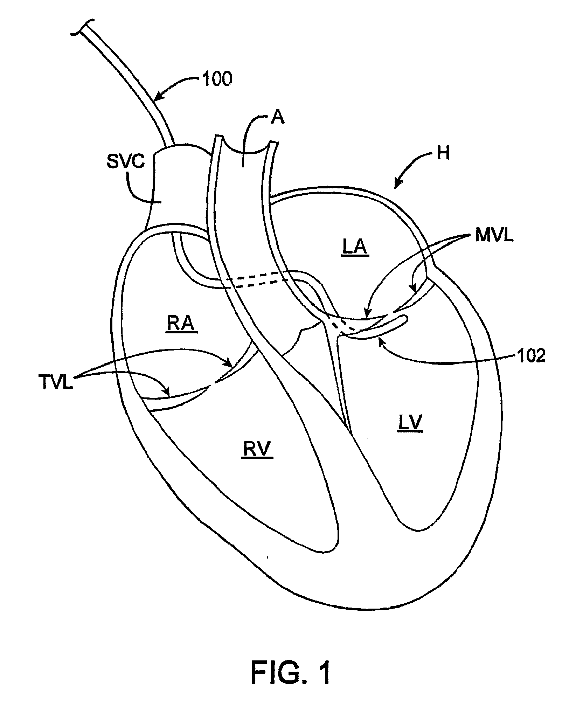

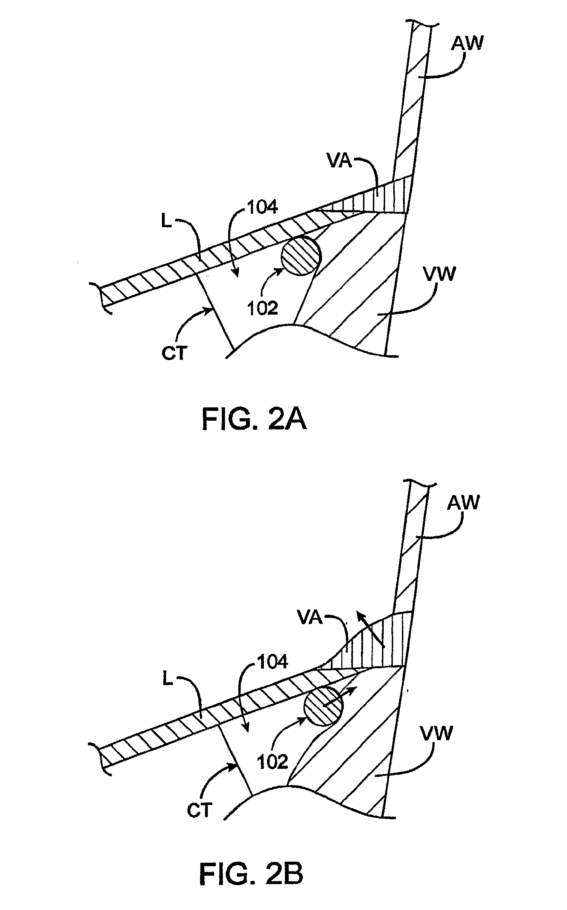

Referring now to Figure 1, a heart H is shown in cross section, with an elongate anchor delivery device 100 introduced within the heart H. Generally, delivery device 100 comprises an elongate body with a distal portion 102 configured to deliver anchors to a heart valve annulus. (In Figs. 1, 2A and 2B, distal portion 102 is shown diagrammatically without anchors or anchor-delivery mechanism to enhance clarity of the figures.) In some embodiments, the elongate body comprises a rigid shaft, while in other embodiments it comprises a flexible catheter, so that distal portion 102 may be positioned in the heart H and under one or more valve leaflets to engage a valve annulus via a transvascular approach. Transvascular access may be gained, for example, through the internal jugular vein (not shown) to the superior vena cava SVC to the right atrium RA, across the interatrial septum to the left atrium LA, and then under one or more mitral valve leaflets MVL to a position within the left ventricle (LV) under the valve annulus (not shown). Alternatively, access to the heart may be achieved via the femoral vein and the inferior vena cava. In other embodiments, access may be gained via the coronary sinus (not shown) and through the atrial wall into the left atrium. In still other embodiments, access may be achieved via a femoral artery and the aorta, into the left ventricle, and under the mitral valve. This access route will be described in further detail below. Any other suitable access route is also contemplated within the scope of the present invention.

In other embodiments, access to the heart H may be transthoracic, with delivery device 100 being introduced into the heart via an incision or port on the heart wall. Even open heart surgical procedures may benefit from methods and devices of the invention. Furthermore, some embodiments may be used to enhance procedures on the tricuspid valve annulus, adjacent the tricuspid valve leaflets TVL, or any other cardiac or vascular valve. Therefore, although the following description typically focuses on minimally invasive or less invasive mitral valve repair for treating mitral regurgitation, the invention is in no way limited to that use.

With reference now to Figures 2A and 2B, a method for positioning delivery device 100 for treating a mitral valve annulus VA is depicted diagrammatically in a cross-sectional view. First, as in Figure 2A, distal portion 102 is positioned in a desired location under a mitral valve leaflet L and adjacent a ventricular wall VW. (Again, distal portion 102 is shown without anchors or anchor-delivery mechanism for demonstrative purposes.) The valve annulus VA generally comprises an area of heart wall tissue at the junction of the ventricular wall VW and the atrial wall AW that is relatively fibrous and, thus, significantly stronger that leaflet tissue and other heart wall tissue.

Distal portion 102 may be advanced into position under the valve annulus by any suitable technique, some of which are described below in further detail. Generally, distal portion 102 may be used to deliver anchors to the valve annulus, to stabilize and/or expose the annulus, or both. In one embodiment, using a delivery device having a flexible elongate body as shown in Figure 1, a flexible distal portion 102 may be passed from the right atrium RA through the interatrial septum in the area of the foramen ovale (not shown--behind the aorta A), into the left atrium LA and thus the left ventricle LV. Alternatively, flexible distal portion 102 may be advanced through the aorta A and into the left ventricle LV, for example using access through a femoral artery. Oftentimes, distal portion 102 will then naturally travel, upon further advancement, under the posterior valve leaflet L into a space defined above a subvalvular space 104 roughly defined for the purposes of this application as a space bordered by the inner surface of the left ventricular wall VW, the inferior surface of mitral valve leaflets L, and cordae tendineae CT connected to the ventricular wall VW and the leaflet L. It has been found that a flexible anchor delivery catheter, such as the delivery devices of the present invention, when passed under the mitral valve via an intravascular approach, often enters subvalvular space 104 relatively easily and may be advanced along space 104 either partially or completely around the circumference of the valve. Once in space 104, distal portion 102 may be conveniently positioned at the intersection of the valve leaflet(s) and the ventricular wall VW, which intersection is immediately adjacent or very near to the valve annulus VA, as shown in Figure 2A. These are but examples of possible access routes of an anchor delivery device to a valve annulus, and any other access routes may be used.

In some embodiments, distal portion 102 includes a shape-changing portion which enables distal portion 102 to conform to the shape of the valve annulus VA. The catheter may be introduced through the vasculature with the shape-changing distal portion in a generally straight, flexible configuration. Once it is in place beneath the leaflet at the intersection between the leaflet and the interior ventricular wall, the shape of distal portion 102 is changed to conform to the annulus and usually the shape is "locked" to provide sufficient stiffness or rigidity to permit the application of force from distal portion 102 to the annulus. Shaping and optionally locking distal portion 102 may be accomplished in any of a number of ways. For example, in some embodiments, a shape-changing portion may be sectioned, notched, slotted or segmented and one of more tensioning members such as tensioning cords, wires or other tensioning devices coupled with the shape-changing portion may be used to shape and rigidify distal portion 102. A segmented distal portion, for example, may include multiple segments coupled with two tensioning members, each providing a different direction of articulation to the distal portion. A first bend may be created by tensioning a first member to give the distal portion a C-shape or similar shape to conform to the valve annulus, while a second bend may be created by tensioning a second member to articulate the C-shaped member upwards against the annulus. In another embodiment, a shaped expandable member, such as a balloon, may be coupled with distal portion 102 to provide for shape changing/deforming. In various embodiments, any configurations and combinations may be used to give distal portion 102 a desired shape.

In transthoracic and other embodiments, distal portion 102 may be shaped, and the method may simply involve introducing distal portion 102 under the valve leaflets. The shaped distal portion 102 may be rigid or formed from any suitable super-elastic or shape memory material, such as nitinol, spring stainless steel, or the like.

In addition to delivering anchors to the valve annulus VA, delivery device 100 (and specifically distal portion 102) may be used to stabilize and/or expose the valve annulus VA. Such stabilization and exposure are described fully in U.S. Patent Application Serial No. 10/656797, which was previously incorporated by reference. For example, once distal portion 102 is positioned under the annulus, force may be applied to distal portion 102 to stabilize the valve annulus VA, as shown in Figure 2B. Such force may be directed in any suitable direction to expose, position and/or stabilize the annulus. For example, upward and lateral force is shown in Figure 2B by the solid-headed arrow drawn from the center of distal portion 102. In other cases, only upward, only lateral, or any other suitable force(s) may be applied. With application of force to distal portion 102, the valve annulus VA is caused to rise or project outwardly, thus exposing the annulus for easier viewing and access. The applied force may also stabilize the valve annulus VA, also facilitating surgical procedures and visualization.

Some embodiments may include a stabilization component as well as an anchor delivery component. For example, some embodiments may include two flexible members, one for contacting the atrial side of a valve annulus and the other for contacting the ventricular side. In some embodiments, such flexible members may be used to "clamp" the annulus between them. One of such members may be an anchor delivery member and the other may be a stabilization member, for example. Any combination and configuration of stabilization and/or anchor delivery members is contemplated.

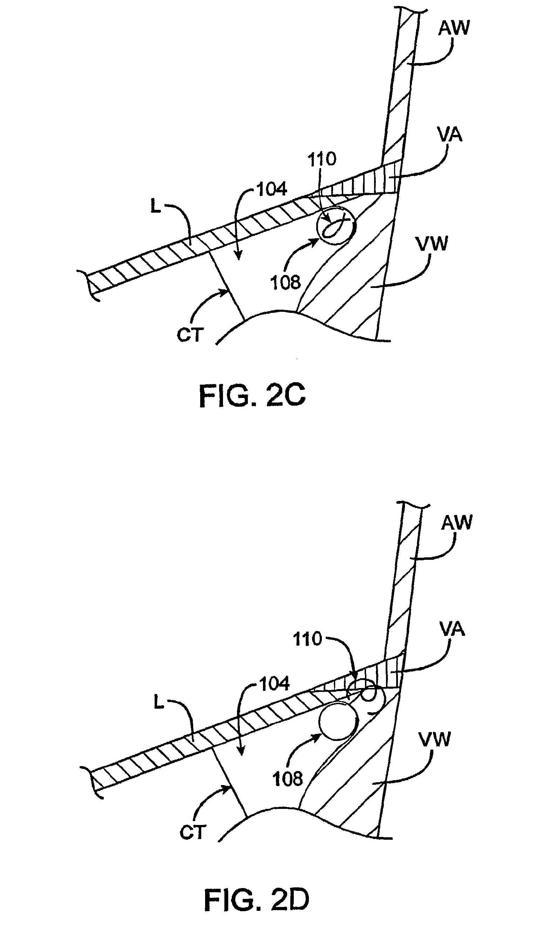

Referring now to Figures 2C and 2D, an anchor delivery device 108 is shown delivering an anchor 110 to a valve annulus VA. Of course, these are again representational figures and are not drawn to scale. Anchor 110 is shown first housed within delivery device 108 (Fig. 2C) and then delivered to the annulus VA (Fig. 2D). As is shown, in one embodiment anchors 110 may have a relatively straight configuration when housed in delivery device 108, perhaps with two sharpened tips and a loop in between the tips. Upon deployment from delivery device 108, the tips of anchor 110 may curve in opposite directions to form two semi-circles, circles, ovals, overlapping helices or the like. This is but one example of a type of self-securing anchor which may be delivered to a valve annulus. Typically, multiple coupled anchors 110 are delivered, and the anchors 110 are drawn together to tighten the valve annulus. Methods for anchor delivery and for drawing anchors together are described further below.

Although delivery device 108 is shown having a circular cross-sectional shape in Figs. 2C and 2D, it may alternatively have any other suitable shape. In one embodiment, for example, it may be advantageous to provide a delivery device having an ovoid or elliptical cross-sectional shape. Such a shape may help ensure that the device is aligned, when positioned between in a corner formed by a ventricular wall and a valve leaflet, such that one or more openings in the delivery device is oriented to deliver the anchors into valve annulus tissue. To further enhance contacting of the valve annulus and/or orientation of the delivery device, some embodiments may further include an expandable member, coupled with the delivery device, which expands to urge or press or wedge the delivery device into the corner formed by the ventricle wall and the leaflet to contact the valve annulus. Such enhancements are described further below.

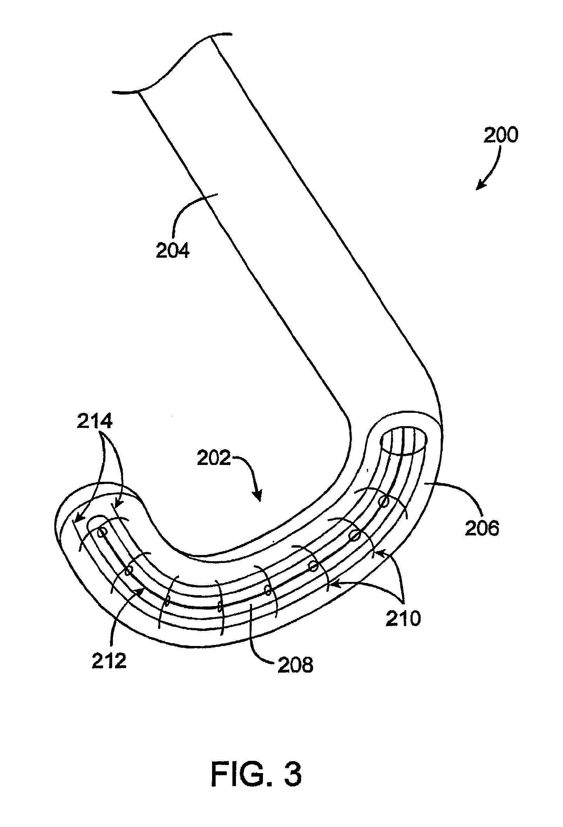

With reference now to Figure 3, one embodiment of a portion of an anchor delivery device 200 suitably includes an elongate shaft 204 having a distal portion 202 configured to deliver a plurality of anchors 210, coupled with a tether 212, to tissue of a valve annulus. Tethered anchors 210 are housed within a housing 206 of distal portion 202, along with one or more anchor retaining mandrels 214 and an expandable member 208. Many variations may be made to one or more of these features, and various parts may be added or eliminated, without departing from the scope of the invention. Some of these variations are described further below, but no specific embodiment(s) should be construed to limit the scope of the invention as defined by the appended claims.

Housing 206 may be flexible or rigid in various embodiments. In some embodiments, for example, flexible housing 206 may be comprised of multiple segments configured such that housing 206 is deformable by tensioning a tensioning member coupled to the segments. In some embodiments, housing 206 is formed from an elastic material having a geometry selected to engage and optionally shape or constrict the valve annulus. For example, the rings may be formed from super-elastic material, shape memory alloy such as Nitinol, spring stainless steel, or the like. In other instances, housing 206 could be formed from an inflatable or other structure can be selectively rigidified *in situ,* such as a gooseneck or lockable element shaft, any of the rigidifying structures described above, or any other rigidifying structure.

"Anchors," for the purposes of this application, is defined to mean any fasteners. Thus, anchors 210 may comprise C-shaped or semicircular hooks, curved hooks of other shapes, straight hooks, barbed hooks, clips of any kind, T-tags, or any other suitable fastener(s). In one embodiment, as described above, anchors may comprise two tips that curve in opposite directions upon deployment, forming two intersecting semi-circles, circles, ovals, helices or the like. In some embodiment, anchors 210 are self-deforming. By "self-deforming" it is meant that anchors 210 change from a first undeployed shape to a second deployed shape upon release of anchors 210 from restraint in housing 206. Such self-deforming anchors 210 may change shape as they are released from housing 206 and enter valve annulus tissue, to secure themselves to the tissue. Thus, a crimping device or other similar mechanism is not required on distal end 202 to apply force to anchors 210 to attach them to annular tissue.

Self-deforming anchors 210 may be made of any suitable material, such as a super-elastic or shape-memory material like Nitinol or spring stainless steel. In other embodiments, anchors 210 may be made of a non-shape-memory material and made be loaded into housing 206 in such a way that they change shape upon release. Alternatively, anchors 210 that are not self-deforming may be used, and such anchors may be secured to tissue via crimping, firing or the like. Even self-securing anchors may be crimped in some embodiments, to provide enhanced attachment to tissue. In some embodiments, anchors 210 may comprise one or more bioactive agent. In another embodiment, anchors 210 may comprise electrodes. Such electrodes, for example, may sense various parameters, such as but not limited to impedance, temperature and electrical signals. In other embodiments, such electrodes may be used to supply energy to tissue at ablation or sub-ablation amounts. Delivery of anchors may be accomplished by any suitable device and technique, such as by simply releasing the anchors by hydraulic balloon delivery as discussed further below. Any number, size and shape of anchors 210 may be included in housing 206.

In one embodiment, anchors 210 are generally C-shaped or semicircular in their undeployed form, with the ends of the C being sharpened to penetrate tissue. Midway along the C-shaped anchor 210, an eyelet may be formed for allowing slidable passage of tether 212. To maintain anchors 210 in their C-shaped, undeployed state, anchors 210 may be retained within housing 206 by two mandrels 214, one mandrel 214 retaining each of the two arms of the C-shape of each anchor 210. Mandrels 214 may be retractable within elongate catheter body 204 to release anchors 210 and allow them to change from their undeployed C-shape to a deployed shape. The deployed shape, for example, may approximate a complete circle or a circle with overlapping ends, the latter appearing similar to a key ring. Such anchors are described further below, but generally may be advantageous in their ability to secure themselves to annular tissue by changing from their undeployed to their deployed shape. In some embodiments, anchors 210 are also configured to lie flush with a tissue surface after being deployed. By "flush" it is meant that no significant amount of an anchor protrudes from the surface, although some small portion may protrude.

Tether 212 may be one long piece of material or two or more pieces and may comprise any suitable material, such as suture, suture-like material, a Dacron strip or the like. Retaining mandrels 214 may also have any suitable configuration and be made of any suitable material, such as stainless steel, titanium, Nitinol, or the like. Various embodiments may have one mandrel, two mandrels, or more than two mandrels.

In some embodiments, anchors 210 may be released from mandrels 214 to contact and secure themselves to annular tissue without any further force applied by delivery device 200. Some embodiments, however, may also include one or more expandable members 208, which may be expanded to help drive anchors 210 into tissue. Expandable member(s) 208 may have any suitable size and configuration and may be made of any suitable material(s). Hydraulic systems such as expandable members are known in the art, and any known or as yet undiscovered expandable member may be included in housing 206 as part of the present invention.

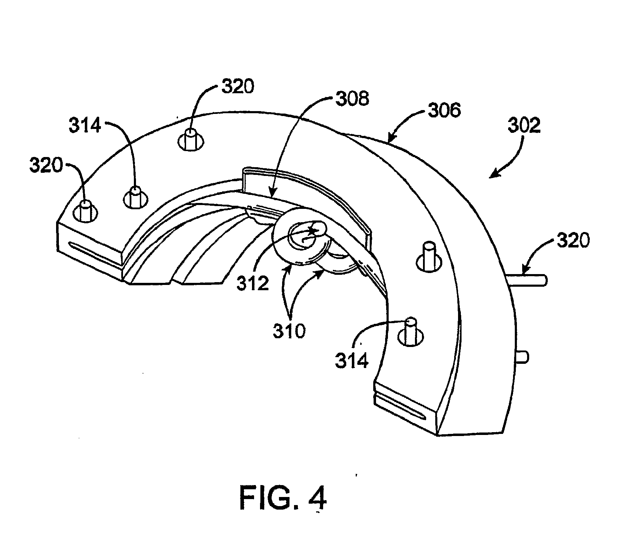

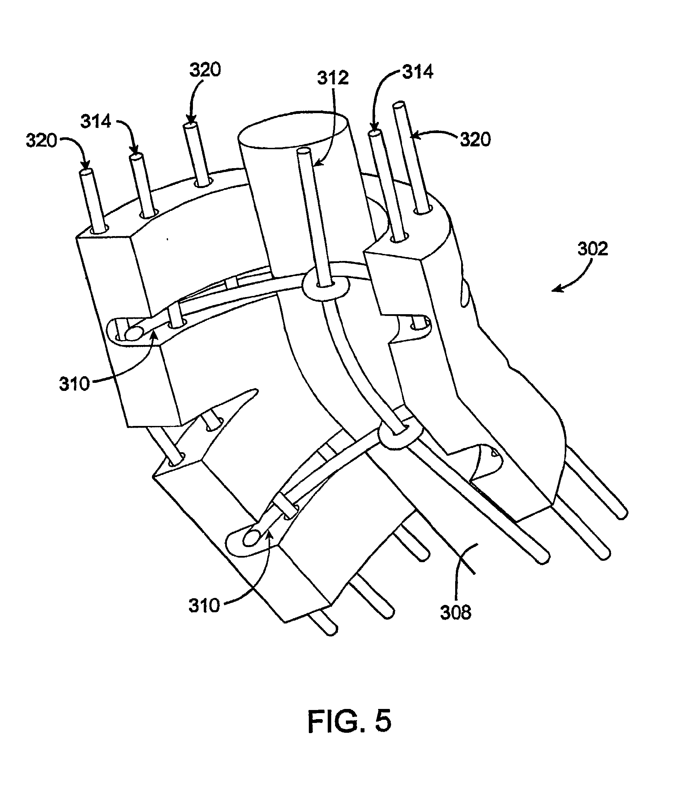

Referring now to Figures 4 and 5, a segment of a distal portion 302 of an anchor delivery device suitably includes a housing 306, multiple tensioning members 320 for applying tension to housing 306 to change its shape, two anchor retaining mandrels 314 slidably disposed in housing 306, multiple anchors 310 slidably coupled with a tether 312, and an expandable member 308 disposed between anchors 310 and housing 306. As can be seen in Figs. 4 and 5, housing 306 may include multiple segments to allow the overall shape of housing 306 to be changed by applying tension to tensioning members 320. As also is evident from the drawings, "C-shaped" anchors 310 may actually have an almost straight configuration when retained by mandrels 314 in housing 306. Thus, for the purposes of this application, "C-shaped" or "semicircular" refers to a very broad range of shapes including a portion of a circle, a slightly curved line, a slightly curved line with an eyelet at one point along the line, and the like.

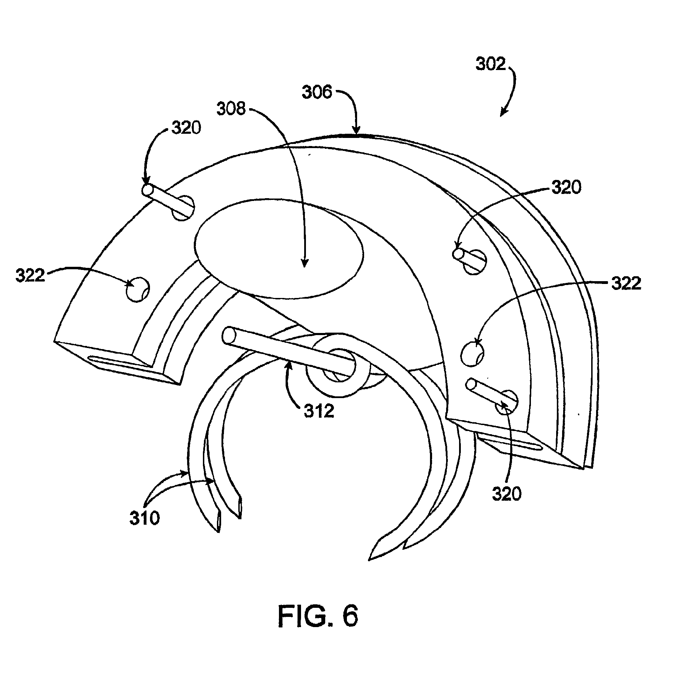

With reference now to Figure 6, the same segment of distal portion 302 is shown, but mandrels 314 have been withdrawn from two mandrel apertures 322, to release anchors 310 from housing 306. Additionally, expandable member 308 has been expanded to drive anchors out of housing 306. Anchors 310, having been released from mandrels 314, have begun to change from their undeployed, retained shape to their deployed, released shape.

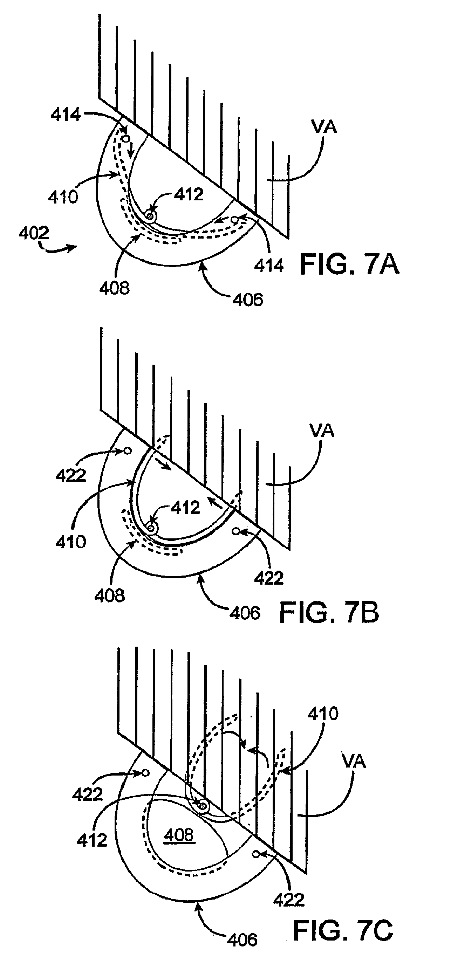

Referring now to Figures 7A-7E, a cross-section of a distal portion 402 of an anchor delivery device is shown in various stages of delivering an anchor to tissue of a valve annulus VA. In Fig. 7A, distal portion 402 is positioned against the valve annulus, an anchor 410 is retained by two mandrels 414, a tether 412 is slidably disposed through an eyelet on anchor 410, and an expandable member 408 is coupled with housing 406 in a position to drive anchor 410 out of housing 406. When retained by mandrels 414, anchor 410 is in its undeployed shape. As discussed above, mandrels 414 may be slidably retracted, as designated by the solid-tipped arrows in Fig. 7A, to release anchor 410. In various embodiments, anchors 410 may be released one at a time, such as by retracting mandrels 414 slowly, may be released in groups, or may all be released simultaneously, such as by rapid retraction of mandrels 414.

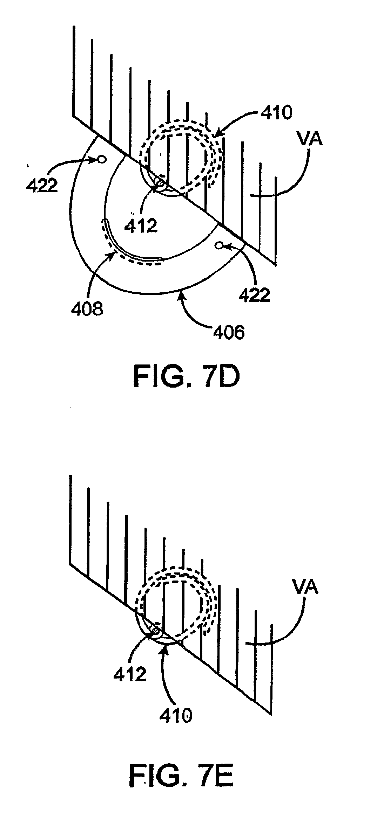

In Fig. 7B, anchor 410 has begun to change from its undeployed shape to its deployed shape (as demonstrated by the hollow-tipped arrows) and has also begun to penetrate the annular tissue VA. Empty mandrel apertures 422 demonstrate that mandrels 414 have been retracted at least far enough to release anchor 410. In Fig. 7B, expandable member 408 has been expanded to drive anchor 410 partially out of housing 406 and further into the valve annulus VA. Anchor 410 also continues to move from its undeployed towards its deployed shape, as shown by the hollow-tipped arrows. In Fig. 7D, anchor 410 has reached its deployed shape, which is roughly a completed circle with overlapping ends or a "key ring" shape. In Fig. 7E, delivery device 402 has been removed, leaving a tethered anchor in place in the valve annulus. Of course, there will typically be a plurality of tethered anchors secured to the annular tissue. Tether 412 may then be cinched to apply force to anchors 410 and cinch and tighten the valve annulus.

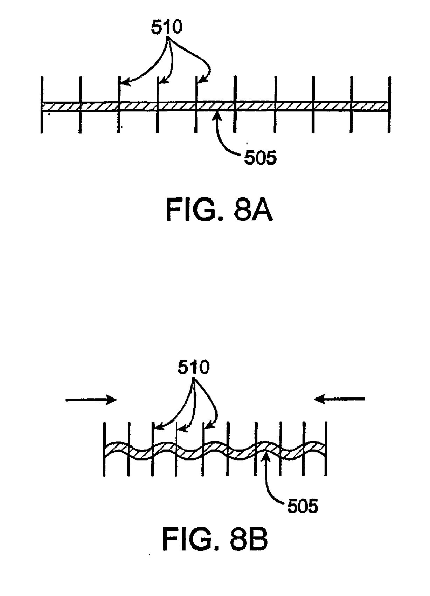

With reference now to Figures 8A and 8B, a diagrammatic representation of another embodiment of coupled anchors is shown. Here, anchors 510 are coupled to a self-deforming or deformable coupling member or backbone 505. Backbone 505 may be fabricated, for example, from Nitinol, spring stainless steel, or the like, and may have any suitable size or configuration. In one embodiment, as in Fig. 8A, backbone 505 is shaped as a generally straight line when held in an undeployed state, such as when restrained within a housing of an anchor deliver device. When released from the delivery device, backbone 505 may change to a deployed shape having multiple bends, as shown in Fig. 8B. By bending, backbone 505 shortens the longitudinal distance between anchors, as demonstrated by the solid-tipped arrows in Fig. 8B. This shortening process may act to cinch a valve annulus into which anchors 510 have be secured. Thus, anchors 510 coupled to backbone 505 may be used to cinch a valve annulus without using a tether or applying tethering force. Alternatively, a tether may also be coupled with anchors 510 to further cinch the annulus. In such an embodiment, backbone 505 will be at least partially conformable or cinchable, such that when force is applied to anchors 510 and backbone 505 via a tether, backbone 505 bends further to allow further cinching of the annulus.

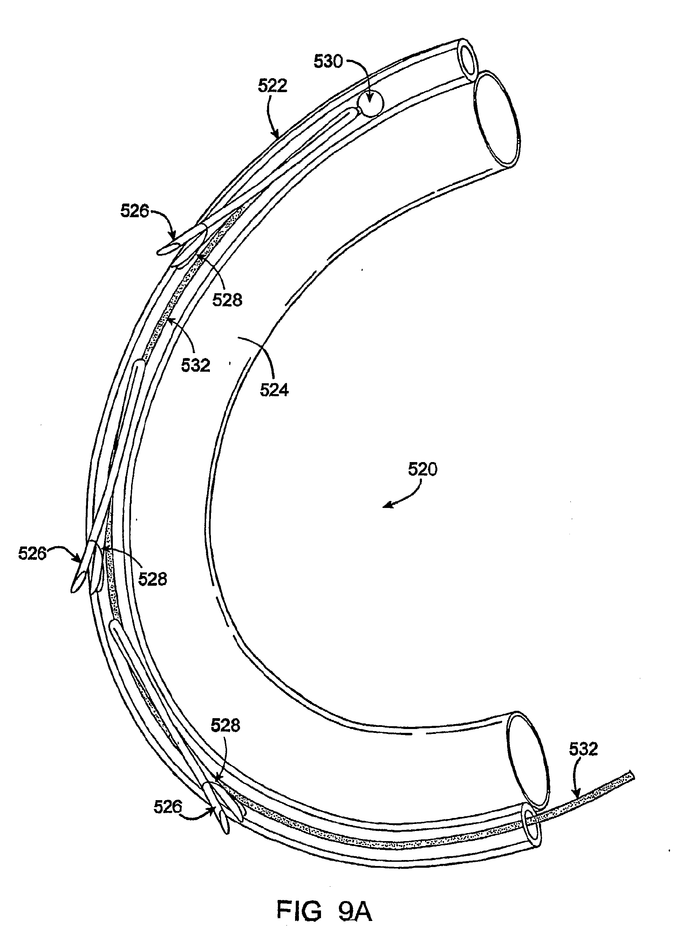

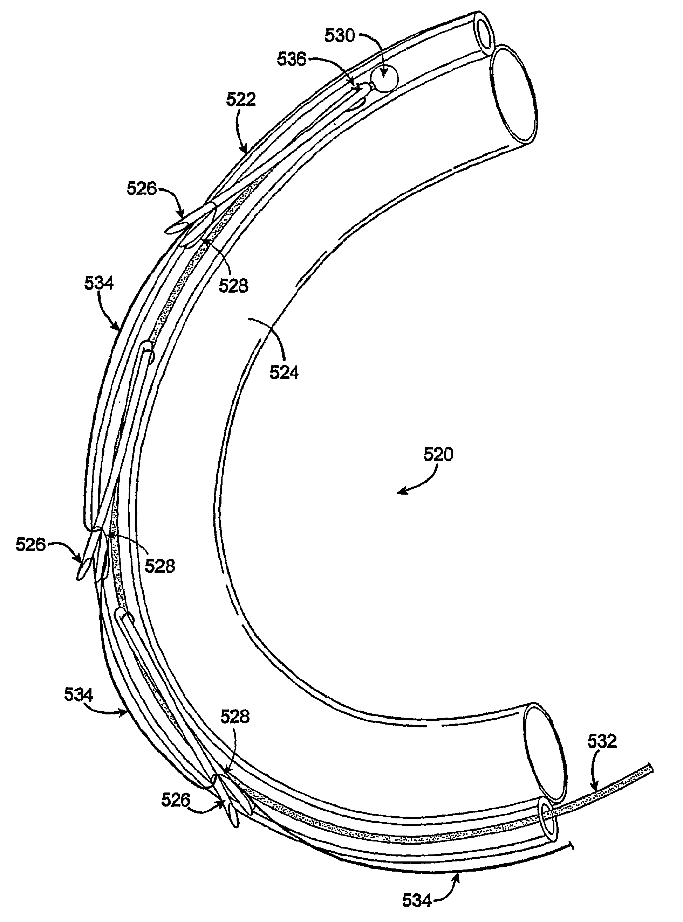

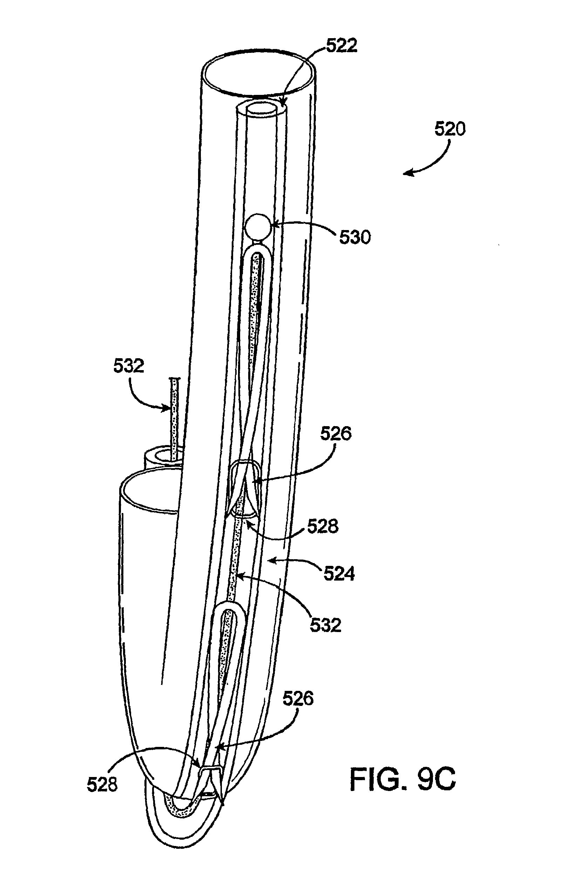

Referring now to Figures 9A-9C, in one embodiment a flexible distal portion of an anchor delivery device 520 suitably includes a housing 522 coupled with an expandable member 524. Housing 522 may be configured to house multiple coupled anchors 526 and an anchor contacting member 530 coupled with a pull cord 532. Housing 522 may also include multiple apertures 528 for allowing egress of anchors 526. For clarity, delivery device 520 is shown without a tether in Figures 9A and 9C, but Figure 9B shows that a tether 534 may extend through an eyelet, loop or other portion of each anchor 526, and may exit each aperture 528 to allow for release of the plurality of anchors 526. The various features of this embodiment are described further below.

In the embodiment shown in Figures 9A-9C, anchors 526 are relatively straight and lie relatively in parallel with the long axis of delivery device 522. Anchor contacting member 530, which may comprise any suitable device, such as a ball, plate, hook, knot, plunger, piston, or the like, generally has an outer diameter that is nearly equal to or slightly less than the inner diameter of housing 522. Contacting member 530 is disposed within the housing, distal to a distal-most anchor 526, and is retracted relative to housing 522 by pulling pull cord 532. When retracted, anchor contacting member 530 contacts and applies force to a distal-most anchor 526 to release cause that anchor 526 to exit housing 522 via one of the apertures 528. Contacting member 530 is then pulled farther proximally to contact and apply force to the next anchor 526 to deploy that anchor 526, and so on.

Retracting contacting member 530 to push anchors 526 out of apertures 528 may help cause anchors 526 to avidly secure themselves to adjacent tissue. Using anchors 526 that are relatively straight/flat when undeployed allows anchors 526 with relatively large deployed sizes to be disposed in (and delivered from) a relatively small housing 522. In one embodiment, for example, anchors 526 that deploy into a shape approximating two intersecting semi-circles, circles, ovals, helices, or the like, and that have a radius of one of the semi-circles of about 3 mm may be disposed within a housing 522 having a diameter of about 5 French (1.67 mm) and more preferably 4 French (1.35 mm) or even smaller. Such anchors 526 may measure about 6 mm or more in their widest dimension. In some embodiments, housing 522 may have a diametrical dimension ("d") and anchor 526 may have a diametrical dimension ("D") in the deployed state, and the ratio of D to d may be at least about 3.5. In other embodiments, the ratio of D to d may be at least about 4.4, and more preferably at least about 7, and even more preferably at least about 8.8. These are only examples, however, and other larger or smaller anchors 526 may be disposed within a larger or smaller housing 522. Furthermore, any convenient number of anchors 526 may be disposed within housing 522. In one embodiment, for example, housing 522 may hold about 1-20 anchors 526, and more preferably about 3-10 anchors 526. Other embodiments may hold more anchors 526.

Anchor contacting member 530 and pull cord 532 may have any suitable configuration and may be manufactured from any material or combination of materials. In alternative embodiments, contacting member 530 may be pushed by a pusher member to contact and deploy anchors 526. Alternatively, any of the anchor deployment devices and methods previously described may be used.

Tether 534, as shown in Figure 9B, may comprise any of the tethers 534 or tether-like devices already described above, or any other suitable device. Tether 534 is generally attached to a distal-most anchor 526 at an attachment point 536. The attachment itself may be achieved via a knot, weld, adhesive, or by any other suitable attachment means. Tether 234 then extends through an eyelet, loop or other similar configuration on each on each of the anchors 526 so as to be slidably coupled with the anchors 526. In the embodiment shown, tether 534 exits each aperture 528, then enters the next-most-proximal aperture, passes slidably through a loop on an anchor 526, and exits the same aperture 528. By entering and exiting each aperture 528, tether 534 allows the plurality of anchors 526 to be deployed into tissue and cinched. Other configurations of housing 522, anchors 526 and tether 534 may alternatively be used. For example, housing 522 may include a longitudinal slit through which tether 534 may pass, thus allowing tether 534 to reside wholly within housing before deployment.

Expandable member 524 is an optional feature of anchor delivery device 520, and thus may be included in some embodiments and not in others. In other words, a distal portion of anchor delivery device 520 may include housing, contents of housing, and other features either with or without an attached expandable member. Expandable member 524 may comprise any suitable expandable member currently known or discovered in the future, and any method and substance(s) may be used to expand expandable member 524. Typically, expandable member 524 will be coupled with a surface of housing 522, will have a larger radius than housing 522, and will be configured such that when it is expanded as housing 522 nears or contacts the valve annulus, expandable member 524 will push or press housing 522 into enhanced contact with the annulus. For example, expandable member 524 may be configured to expand within a space near the corner formed by a left ventricular wall and a mitral valve leaflet.

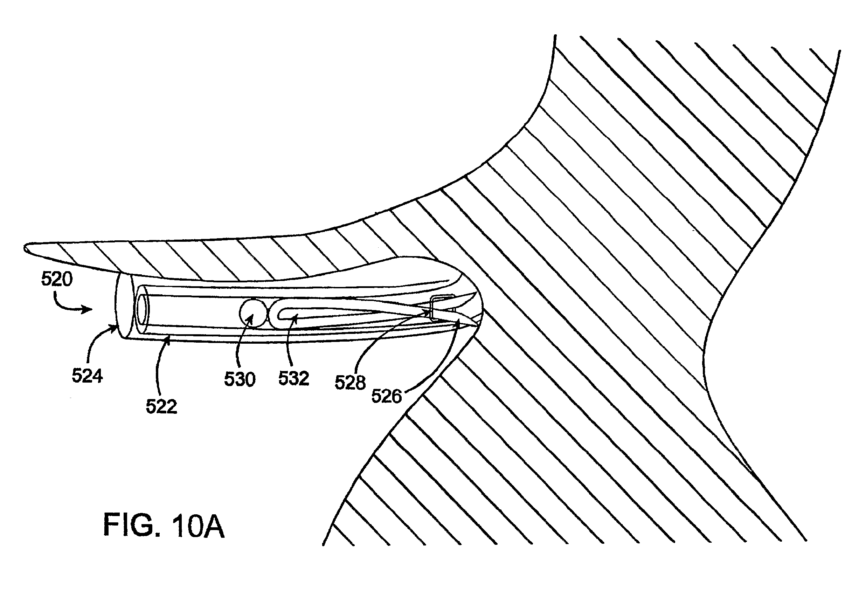

With reference now to Figures 10A-10F, a method is shown for applying a plurality of tethered anchors 526 to a valve annulus VA in a heart. As shown in Figure 10A, an anchor delivery device 520 is first contacted with the valve annulus VA such that openings 528 are oriented to deploy anchors 526 into the annulus. Such orientation may be achieved by any suitable technique. In one embodiment, for example, a housing 522 having an elliptical cross-sectional shape may be used to orient openings 528. As just described, contact between housing 522 and the valve annulus VA may be enhanced by expanding expandable member 524 to wedge housing within a corner adjacent the annulus.

Generally, delivery device 520 may be advanced into any suitable location for treating any valve by any suitable advancing or device placement method. Many catheter-based, minimally invasive devices and methods for performing intravascular procedures, for example, are well known, and any such devices and methods, as well as any other devices or method later developed, may be used to advance or position delivery device 520 in a desired location. For example, in one embodiment a steerable guide catheter is first advanced in retrograde fashion through an aorta, typically via access from a femoral artery. The steerable catheter is passed into the left ventricle of the heart and thus into the space formed by the mitral valve leaflets, the left ventricular wall and cordae tendineae of the left ventricle. Once in this space, the steerable catheter is easily advanced along a portion (or all) of the circumference of the mitral valve. A sheath is advanced over the steerable catheter within the space below the valve leaflets, and the steerable catheter is removed through the sheath. Anchor delivery device 520 may then be advanced through the sheath to a desired position within the space, and the sheath may be removed. In some cases, an expandable member coupled to delivery device 520 may be expanded to wedge or otherwise move delivery device 520 into the corner formed by the left ventricular wall and the valve leaflets to enhance its contact with the valve annulus. Of course, this is but one exemplary method for advancing delivery device 520 to a position for treating a valve, and any other suitable method, combination of devices, etc. may be used.

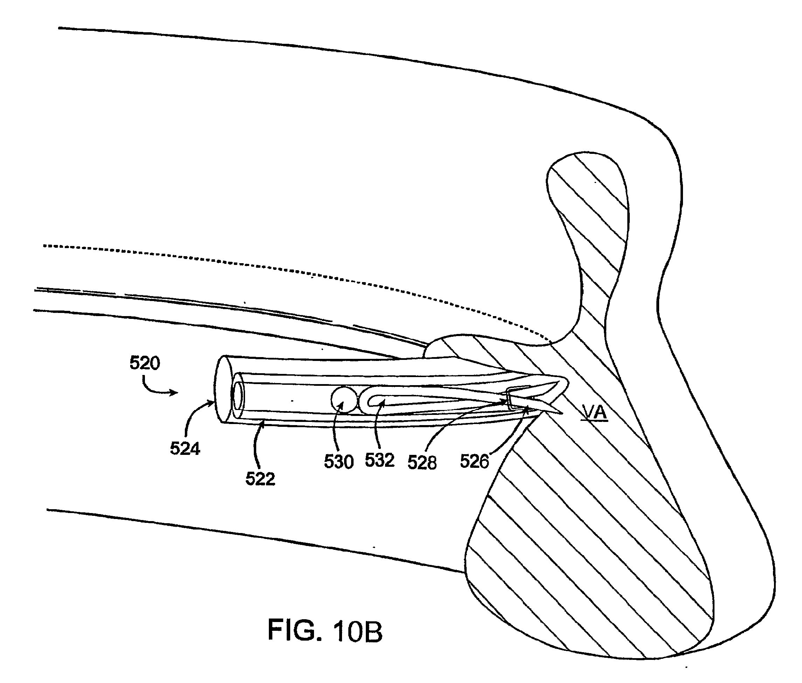

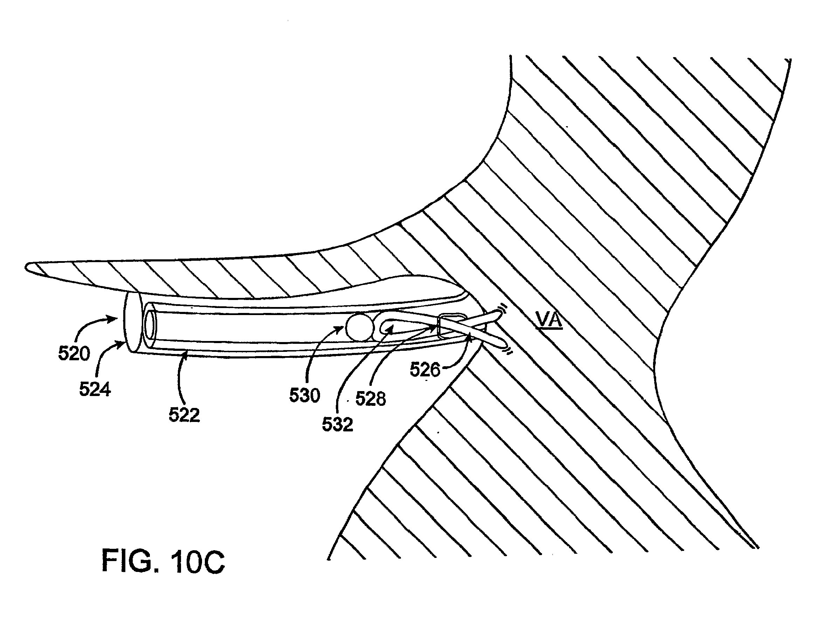

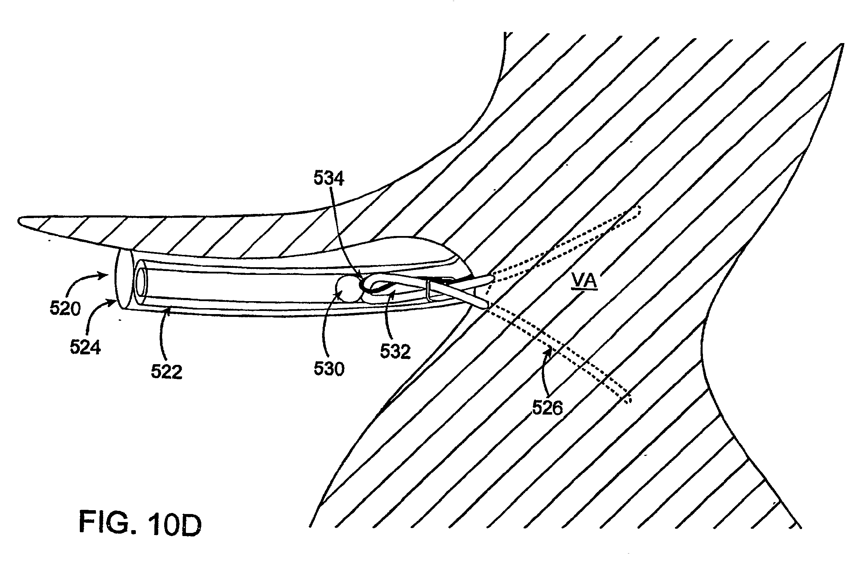

As shown in Figure 10B, when delivery device 520 is positioned in a desired location for deploying anchors 526, anchor contacting member 530 is retracted to contact and apply force to a most-distal anchor 526 to begin deploying anchor 526 through aperture 528 and into tissue of the valve annulus VA. Figure 10C show anchor 526 further deployed out of aperture 528 and into valve annulus VA. Figure 10D shows the valve annulus VA transparently so that further deployment of anchors 526 can be seen. As shown, in one embodiment of the invention, anchors 526 include two sharpened tips that move in opposite directions upon release from housing 522 and upon contacting the valve annulus VA. Between the two sharpened tips, an anchor 526 may be looped or have any other suitable eyelet or other device for allowing slidable coupling with a tether 534.

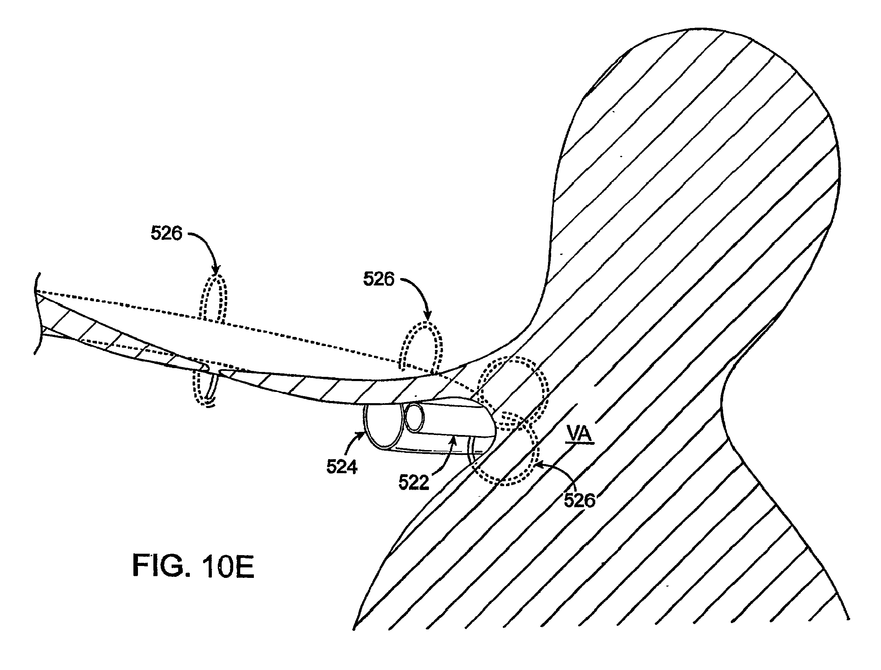

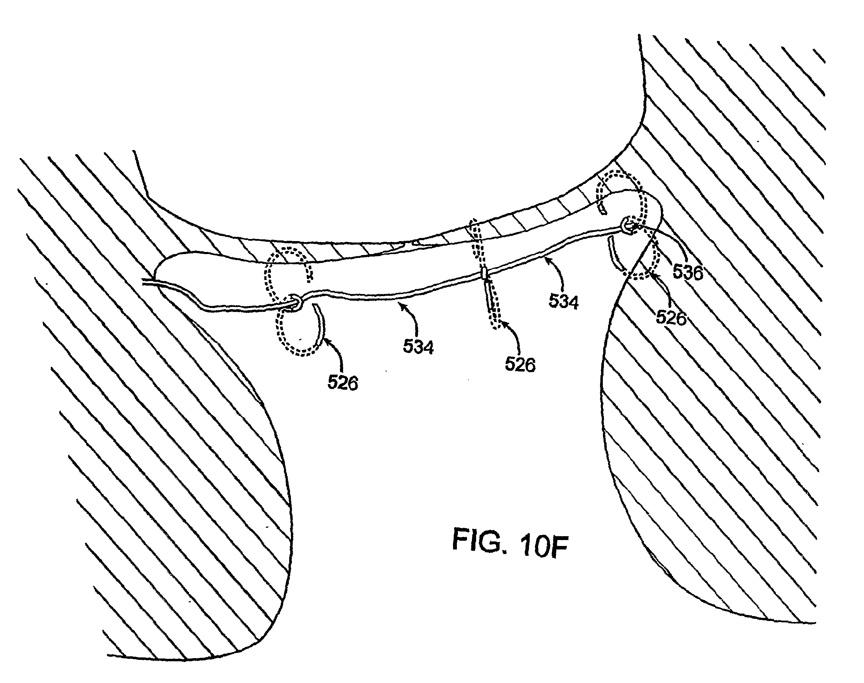

Referring now to Figure 10E, anchors 526 are seen in their fully deployed or nearly fully deployed shape, with each pointed tip (or "arm") of each anchor 526 having curved to form a circle or semi-circle. Of course, in various embodiments anchors 526 may have any other suitable deployed and undeployed shapes, as described more fully above. Figure 10F shows anchors 526 deployed into the valve annulus VA and coupled with tether 534, with the distal-most anchor 526 coupled attached fixedly to tether 524 at attachment point 536. At this stage, tether 534 may be cinched to tighten the annulus, thus reducing valve regurgitation. In some embodiments, valve function may be monitored by means such as echocardiogram and/or fluoroscopy, and tether 534 may be cinched, loosened, and adjusted to achieve a desired amount of tightening as evident via the employed visualization technique(s). When a desired amount of tightening is achieved, tether 534 is then attached to a most-proximal anchor 526 (or two or more most-proximal anchors 526), using any suitable technique, and tether 534 is then cut proximal to the most-proximal anchor 526, thus leaving the cinched, tethered anchors 526 in place along the valve annulus VA. Attachment of tether 534 to the most-proximal anchor(s) 526 may be achieved via adhesive, knotting, crimping, tying or any other technique, and cutting tether 534 may also be performed via any technique, such as with a cutting member coupled with housing 522.

In one embodiment, cinching tether 534, attaching tether 534 to most-proximal anchor 526, and cutting tether 534 are achieved using a termination device (not shown). The termination device may comprise, for example, a catheter advancable over tether 534 that includes a cutting member and a nitinol knot or other attachment member for attaching tether 534 to most-proximal anchor. The termination catheter may be advanced over tether 534 to a location at or near the proximal end of the tethered anchors 526. It may then be used to apply opposing force to the most-proximal anchor 526 while tether 534 is cinched. Attachment and cutting members may then be used to attach tether 534 to most-proximal anchor 526 and cut tether 534 just proximal to most-proximal anchor 526. Such a termination device is only one possible way of accomplishing the cinching, attachment and cutting steps, and any other suitable device(s) or technique(s) may be used.

In some embodiments, it may be advantageous to deploy a first number of anchors 526 along a first portion of a valve annulus VA, cinch the first anchors to tighten that portion of the annulus, move the delivery device 520 to another portion of the annulus, and deploy and cinch a second number of anchors 526 along a second portion of the annulus. Such a method may be more convenient, in some cases, than extending delivery device 520 around all or most of the circumference of the annulus, and may allow a shorter, more maneuverable housing 522 to be used.

In an embodiment similar to that shown in Figs. 10A-10F, an analogous method may be used but anchors 526 may be driven out of delivery device 520 through a biocompatible material attached to delivery device 520, thereby attaching the biocompatible material to the valve annulus VA. For example, in one embodiment a Dacron strip may be attached to delivery device 520, extending along device 520 and covering apertures 528. Anchors 526 are then driven out of delivery device 520, through the Dacron strip, into the valve annulus VA, thus detaching the Dacron strip from device 520 and attaching it to the valve annulus VA. Such a biocompatible material may facilitate tissue ingrowth of anchors 526 and may enhance attachment generally to the valve annulus VA. In an alternative embodiment, multiple pieces of biocompatible material, such as separate pieces of material disposed over each of apertures 528, may be used. For example, in one embodiment multiple discs of Dacron material are disposed over multiple apertures 528.

In another embodiment, a distal portion of delivery device 520 may be detachable from a proximal portion of delivery device 520. Such an embodiment may be configured such that when anchors 526 are deployed from device 520, the distal portion of device 520 detaches from the proximal portion and is attached, via anchors 526, to the valve annulus VA. In one embodiment, for example, anchors 526 may pierce through the distal portion of device 520, rather than exiting device 520 through apertures 528. The distal portion may be detachable via any suitable means, such as perforations or the like.

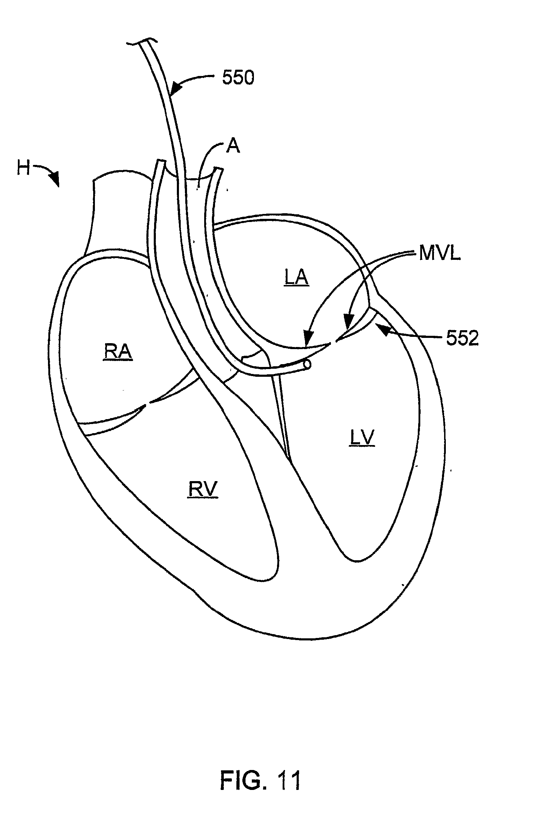

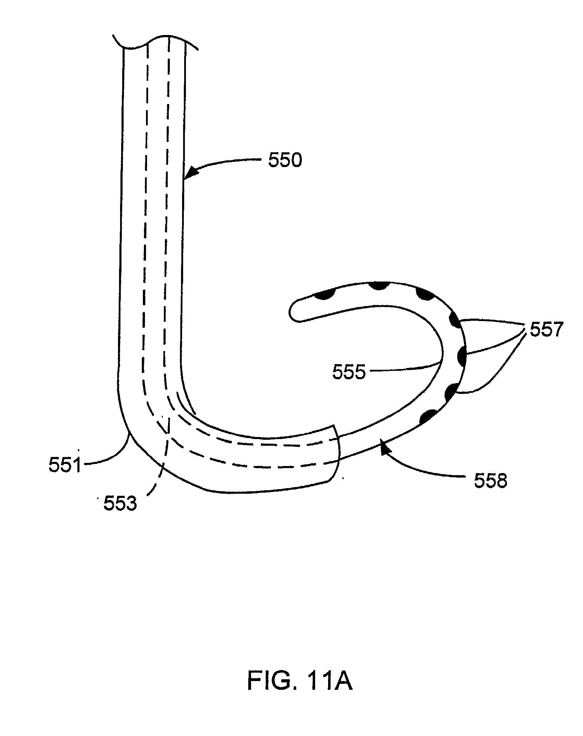

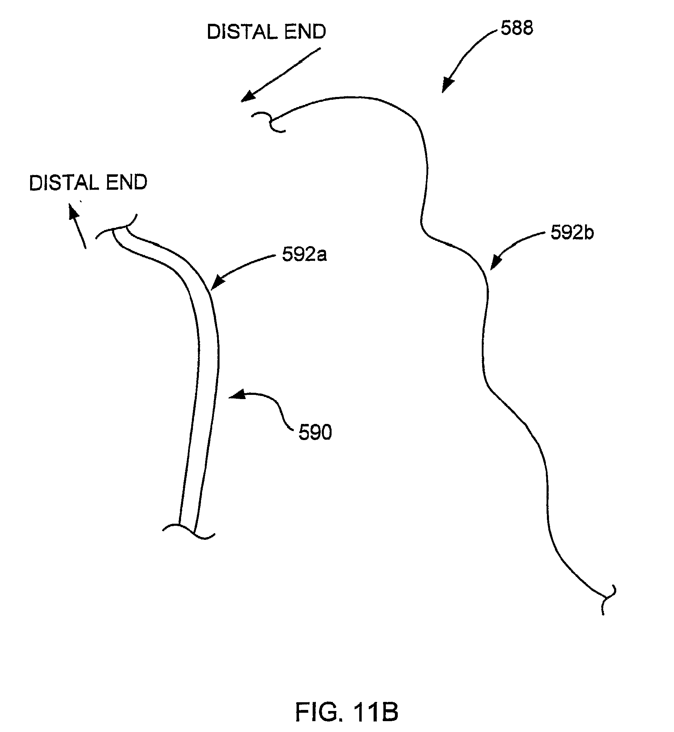

Referring now to Fig. 11, a cross-sectional depiction of a heart H is shown with an anchor delivery device guide catheter 550 advanced through the aorta A and into the left ventricle LV. In a preferred embodiment, this access route to the subannular space and the valve annulus may used. Guide catheter 550 is generally a flexible elongate catheter which may have one or more curves or bends toward its distal end to facilitate placement of the distal end of catheter 550 in a subannular space 552. Subannular space 552, which has been described above in detail, is generally defined by the left ventricular wall, the mitral valve leaflets MVL, and cordae tendiniae, and travels along most or all of the circumference of the valve annulus. The distal end of guide catheter 550 may be configured to be positioned at an opening into space 552 or within space 552, such that subsequent catheter devices may be passed through guide catheter 550 into space 552. In some embodiments, it may be advantageous to provide guide catheter 550 with a curvable portion with a radius in an expanded/curved state that is greater than a radius of the valve annulus. For example, in one embodiment guide catheter 550 in the expanded state has a radius about 25%-50% larger that the valve annulus.

With reference now to Fig. 11A, a distal portion of guide catheter 550 is shown, with an anchor delivery device 558 extending through it and out of its distal end. As shown, in one embodiment guide catheter 550 includes at least one bend 551 or curvature, and anchor delivery device 558 is pre-shaped to include at least one corresponding bend 553, that has approximately the same radius of curvature as the bend 551 in guide catheter 550. In some embodiments (not shown), guide catheter 550 may have multiple bends 551, and anchor delivery device 558 may have multiple corresponding bends 553. In the embodiment shown, anchor delivery device 558 includes a proximal bend 553, which corresponds to the bend 551 in guide catheter 550, and a distal bend 555. By matching the radii of curvature of the proximal bend 553 and the guide catheter bend 551, the distal portion of anchor delivery device 558 becomes automatically oriented (when advanced through guide catheter 550) such that one or more anchor delivery apertures 557 are in contact with the valve annulus (not shown). Moreover, distal bend 555 may have a radius of curvature that matches approximately a radius of curvature of a valve annulus. Alternatively, distal bend 555 may have a radius of curvature greater than a valve annulus radius of curvature, such that the distal portion of anchor delivery device 558 tends to push radially outward, enhancing contact of the device 558 with valve annulus tissue. Such greater radii of curvature are described in greater detail below. Proximal bend 553 and distal bend 555 may therefore have any suitable angles relative to one another and relative to the more proximal portion of anchor delivery device 558. In some embodiments, anchor delivery device 558 is also steerable.

With reference now to Fig. 11B, in the embodiment described immediately above and/or in alternative embodiments, an anchor delivery device 588 and a guide catheter 590 may include one or more corresponding (or "registering") bends or orientation portions 592a, 592b at other locations along their lengths. In other words, although bends 551, 553, 555 are shown in Fig. 11A at or near the distal ends of guide catheter 550 and anchor delivery device 558, similar bends could be formed at more proximal locations. For example, Fig. 11B shows guide catheter 590 with orientation portion 592a having a chosen shape when relaxed. The chosen shape may lie along a two-dimensional or three-dimensional path. Anchor delivery device 588 has a corresponding orientation portion 592b along its length which is complementary to the shape of orientation portion 592a. The chosen shape may also be created by the application of energy, mechanical manipulation or the like. Such orientation portions 592a, 592b could be used for further registering or orienting delivery device 588 to a desired orientation. Typically, when orientation portions 592a, 592b are axially aligned, which can be indicated by orientation markers at the proximal ends of guide catheter 590 and anchor delivery device 588 external of the patient, proper rotary orientation can be sensed tactically by the physician to help insure the distal end of anchor delivery device 588 is properly oriented. Delivery device 588 may be rotated, advanced or moved in any suitable fashion within guide catheter 590 to achieve a desired orientation. The use of one or more complementary orientation portions 592a, 592b may be used with any of a number of various embodiments of guide catheters and anchor delivery devices.

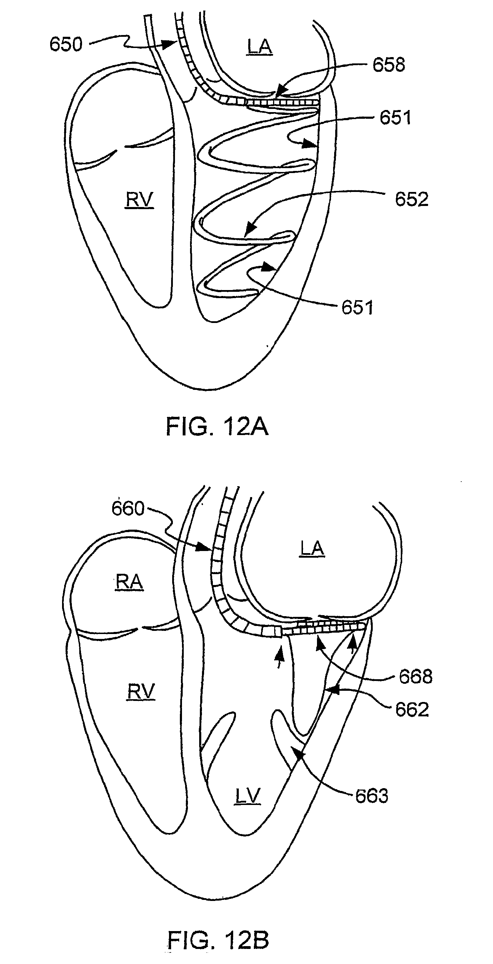

In a number of cases, and with reference now to Figs. 12A-12D, it may be advantageous to provide further support to an anchor delivery device 658, to support the device 658 against valve annulus tissue and/or to push the device 658 against valve annulus tissue to enhance contact with, and anchor delivery into, the tissue. In one embodiment, as shown in Fig. 12A, a helical support member 652 may be coupled with a distal end of anchor delivery device 658 and may be extended into the left ventricle of a heart (or other heart chamber in other embodiments) to contact the heart wall 651 and thus support anchor delivery device 658 against the valve annulus tissue. In alternative embodiments, helical support member 651 may extend out of a guide catheter 650 to contact the heart wall 651 and support anchor delivery device 658. Any suitable means may be used for extending helical member 652 into the left ventricle or other chamber. For example, helical member 652 is pushed out of guide catheter 650 in one embodiment, but may alternatively be extended out of anchor delivery device 658. Helical member 652 may be made of any suitable material, such as but not limited to Nitinol, stainless steel or the like.

In an alternative embodiment, pictured in Fig. 12B, a deployable U-shaped support member 662 may be movably coupled with a distal portion of an anchor delivery device 668, both of which are advanceable through a guide catheter 660. Upon being advanced out of the distal end of guide catheter 660, U-shaped member 662 may automatically spring out, or alternatively may be manually manipulated to extend outward, to contact the inner surface of the heart wall and/or to contact a papillary muscle 663. As shown in Fig. 12B, in one embodiment U-shaped member 663 contacts an intersection of a papillary muscle 663 with the heart wall, and thus provides upward support (solid-tipped arrows) to anchor delivery device 668. Again, such a U-shaped member 662 may automatically deform from a straight configuration for delivery through guide catheter 660 into a U-shaped configuration, such as if member 662 is made of Nitinol, spring stainless steel, or other shape memory or super-elastic material. Alternatively, U-shaped member 662 may be connected to anchor delivery device 668 at or near the distal end of the device 668 and may be pushed distally to force the U-shaped member 662 to expand into its U-shape. In an alternative embodiment, U-shaped member 662 may be attached proximally and may be pulled into its expanded configuration. Any suitable method for changing the shape of U-shaped member 662 from straight to U-shaped may be used in various embodiments.

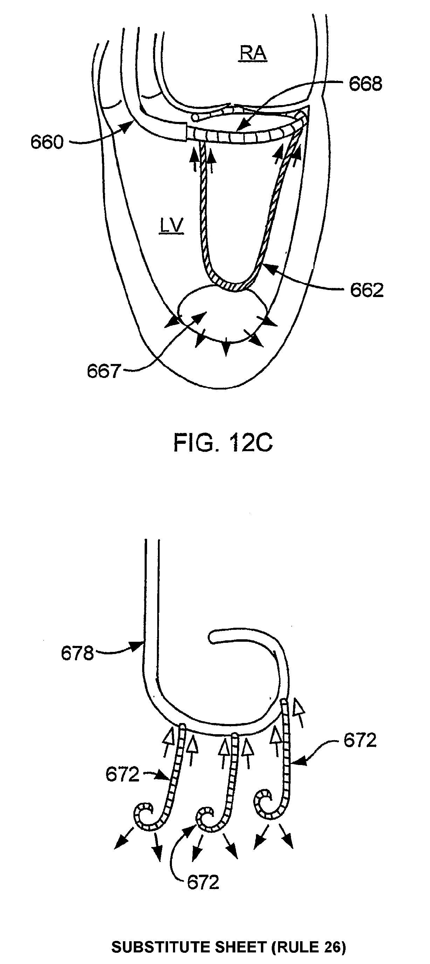

As shown in Fig. 12C, U-shaped member 662 may optionally include an expandable member 667, such as an inflatable balloon. Expandable member 667 may be expanded to provide further force against and support of anchor delivery device 668, to enhance its contact with valve annulus tissue. In another embodiment, as shown in Fig. 12D, multiple spring members 672 may be coupled with a distal end of an anchor delivery device 678 to provide force against an inner surface of a heart wall (solid tipped arrows) to thus support anchor delivery device 678 against annulus tissue (hollow tipped arrows). Thus, various embodiments of the invention may include any of a number of suitable support devices for enhancing support of an anchor delivery device against valve annulus tissue, thus enhancing the ability of the delivery device to delivery tissue anchors into the annulus.

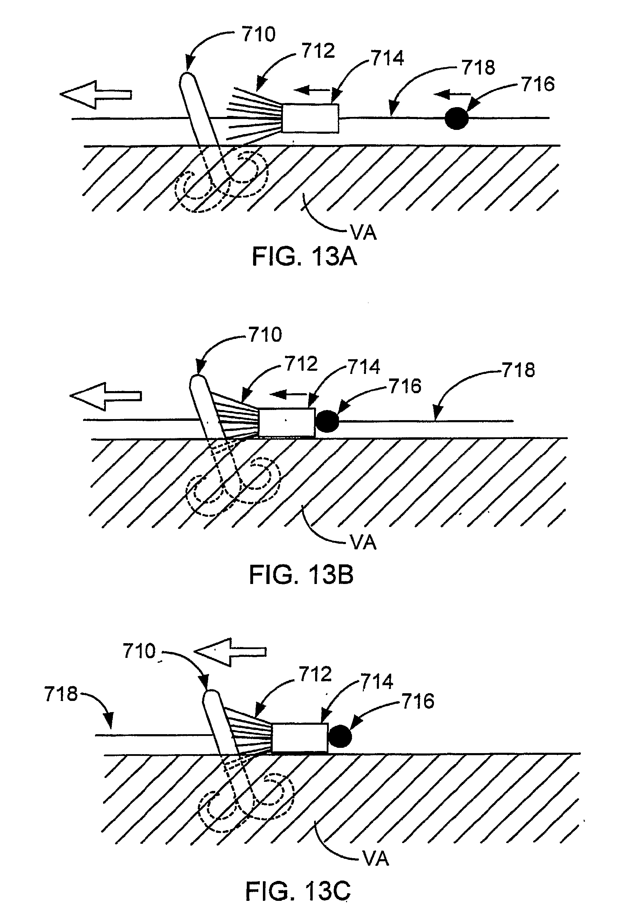

Referring now to Figs. 13A-13C, in some embodiments it may be advantageous to provide one or more devices to enhance the attachment of a terminal tissue anchor 710 to valve annulus tissue VA. Typically, in attaching tissue anchors to valve annulus tissue VA, a first tethered anchor (not shown) is attached, and subsequent anchors are then attached, ending in a final or terminal anchor 710. A tether 718 is then cinched, to apply force between the attached anchors (hollow arrow), thus cinching the valve annulus VA. Tether 718 is then typically attached by any suitable means to terminal anchor 710 and then cut or otherwise detached proximal to the terminal anchor 710, leaving the cinched, tethered anchors in place, attached to the valve annulus VA. To relieve some of the tension placed on terminal anchor 710 and/or to provide additional attachment/anchoring strength to the terminal end of the tethered anchors, one or more locking members 714 may be deployed at or near the terminal end. For example, in one embodiment locking member 714 comprises a cylinder slidably disposed over tether 718, with prongs 712 extending from one end of the cylinder. Locking member 714 is deployed out of the distal end of a termination catheter, guide catheter or the like (not shown) and is then slid along tether 718, such that prongs 712 contact and enter into valve annulus tissue VA. In one embodiment, a pusher member 716, such as a ball slidably disposed over tether 718, may be used to push locking member 714 forward and into engagement with tissue, as shown in Fig. 13B and as designated by solid tipped arrows. In some embodiments, locking member 714 engages with terminal anchor 710, as shown in Figs. 13B and 13C, though such engagement is not required. Once locking member 714 is fully engaged with valve tissue VA, tether 718 is cut proximal to locking member 714. In some embodiments, pusher member 716 remains in place, while in others it may be removed before cutting tether 718.

A number of different variations of locking members are contemplated in various embodiments. For example, a two-pronged member may be used, with the prongs deployable from a delivery position to and expanded configuration, and with the prongs optionally engaging with the terminal anchor 710. In another embodiment, multiple prongs may be aligned in a linear fashion along a locking member, such as in a rake-like configuration. Yet another embodiment include two prongs for engaging with the terminal anchor 710 and another prong for engaging with valve annulus tissue VA. Thus, any of a number of different embodiments may be employed as part of the present invention. Such locking members may be constructed from any suitable material or combination of materials, such as Nitinol, spring stainless steel and/or other shape memory or super-elastic materials.

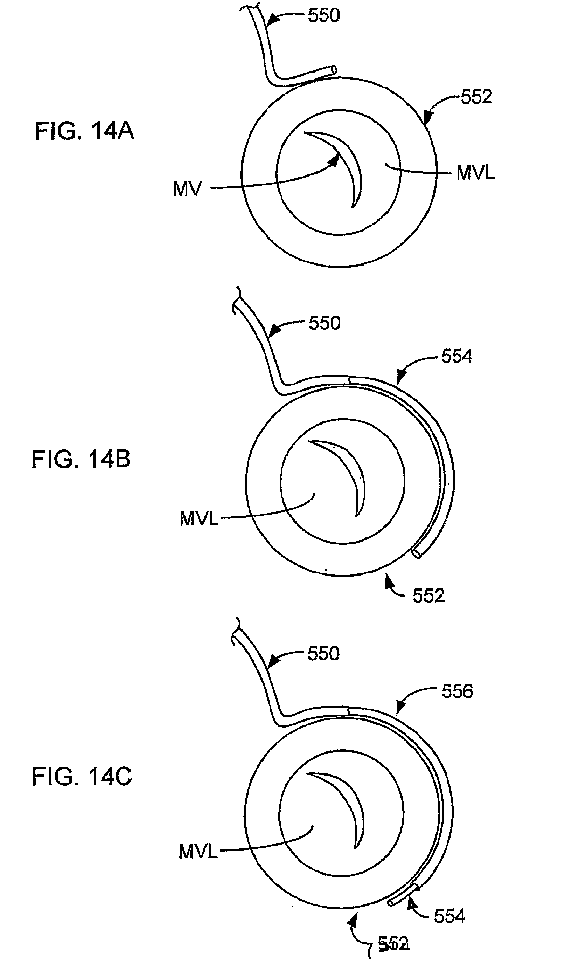

Figs. 14A-14F demonstrate a method for advancing an anchor delivery device to a position for treating a mitral valve MV. The mitral valve MV, including mitral valve leaflets MVL are represented diagrammatically from an inferior perspective looking up, to depict a method for delivering a device into subannular space 552. In Fig. 14A, first guide catheter 550 is show extending up to or into subannular space 552, as in Fig. 11. As shown in Fig. 14B, in one method a second guide catheter 554 may be advanced through first guide catheter 550 to pass through/along subannular space 554. This second guide catheter 554 is steerable in one embodiment, as will be described further below, to help conform second guide catheter 554 to subannular space 552.

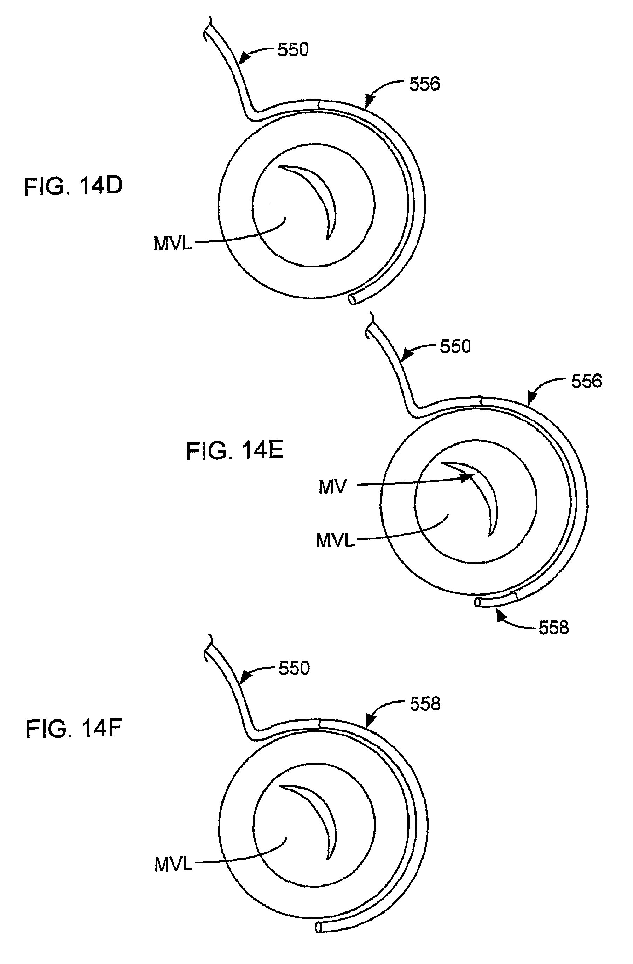

Next, as in Fig. 14C, a guide sheath 556 may be passed over second guide catheter 554 to extend along subannular space. Sheath 556 is generally a flexible, tubular member that can be passed over second guide catheter 554 and within first guide catheter 550. To enhance passage and exchange, any of these and other described catheter members, sheath members, or the like may be manufactured from and/or coated with one or more friction resistant materials. Once sheath 556 is in place, second guide catheter 554 may be withdrawn, as shown in Fig. 14D. As shown in Fig. 14E, an anchor delivery device 558 may then be advanced through sheath 556 to a position for treating the mitral valve MV. Sheath 556 may then be withdrawn, as in Fig. 14F, leaving anchor delivery device 558 in place for performing a treatment. A valve annulus treatment may be performed, as described extensively above, and anchor delivery device 558 may be withdrawn. In some embodiments, anchor delivery device 558 is used to treat one portion of the valve annulus and is then moved to another portion, typically the opposite side, to treat the other portion of the annulus. In such embodiments, any one or more of the steps just described may be repeated. In some embodiments, anchor delivery device 558 is withdrawn through first guide catheter 550, and first guide catheter 550 is then withdrawn. In alternative embodiments, first guide catheter 550 may be withdrawn before anchor delivery device 558.

In various embodiments, alternative means may be used to urge anchor delivery device 558 into contact with the valve annulus. For example, in one embodiment an expandable member is coupled with anchor delivery device 558 and expanded within the subannular space 552. In an alternative embodiment, a magnet may be coupled with anchor delivery device 558, and another anchor may be disposed within the coronary sinus, in proximity to the first magnet. The two magnets may attract one another, thus pulling the anchor delivery device 558 into greater contact with the annulus. In another embodiment, anchor delivery device 558 in an expanded (or deployed) state may have a radius of curvature that is larger than the radius of curvature of the mitral valve annulus, thus causing device 558 to be urged against the annulus. In one embodiment, for example, the radius of curvature of device 558 in the expanded/deployed state is about 25%-50% larger than the radius of curvature of the mitral valve annulus.

Various embodiments may also include visualizing the annulus using a visualization member coupled with the anchor delivery device 558 or separate from the device 558. In some embodiments, anchors may be driven through a strip of detachable, biocompatible material, such as Dacron, that is coupled with anchor delivery device 558 but that detaches to affix to the valve annulus via the anchors. In some embodiments, the strip may then be cinched to tighten the annulus. In other embodiments, the anchors may be driven through a detachable, biocompatible, distal portion of the guide sheath 556, and guide sheath 556 may then remain attached to the annulus via the anchors. Again, in some embodiments, the detached sheath may be cinched to tighten the annulus.

Of course, the method just described is but one embodiment of a method for delivering an anchor delivery device to a location for treating a valve annulus. In various alternative embodiments, one or more steps may be added, deleted or modified while achieving a similar result. In some embodiments, a similar method may be used to treat the mitral valve from a superior/right atrial position or to treat another heart valve. Additionally, other devices or modifications of the system just described may be used in other embodiments.

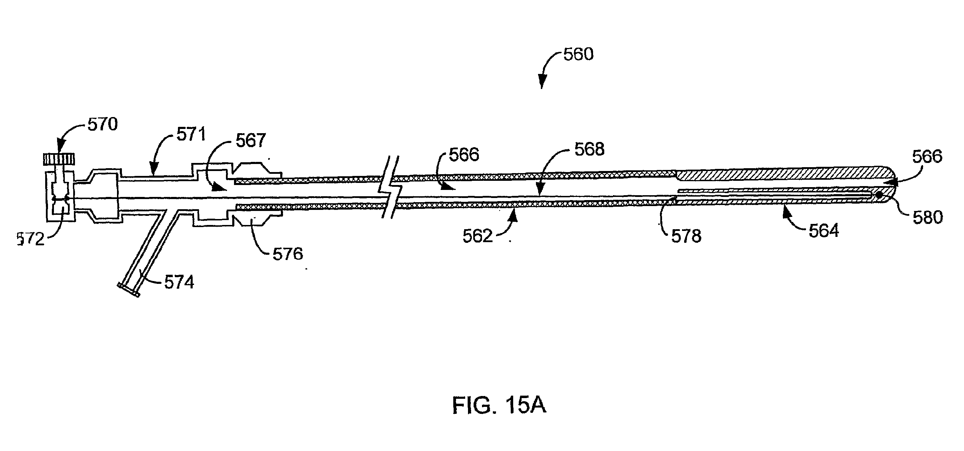

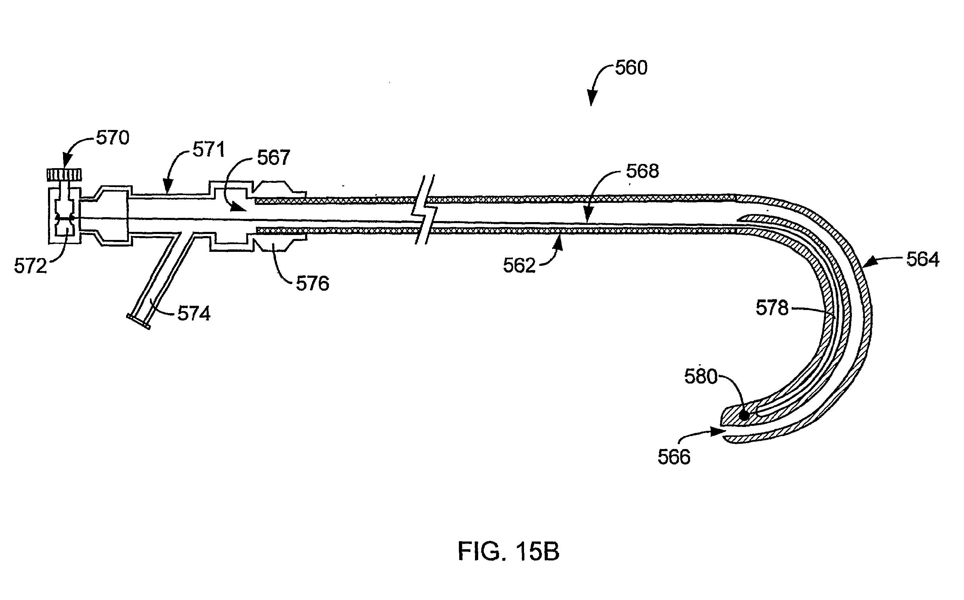

With reference now to Figs. 15A and 15B, one embodiment of a steerable catheter device 560 is shown. Steerable catheter device 560 may be used in a method such as that just described in reference to Figs. 14A-14F, for example in performing a function similar to that performed by second guide catheter 554. In other embodiments, catheter device 560 may perform any other suitable function. As shown, catheter device 560 suitably includes an elongate catheter body having a proximal portion 562 and a distal portion 564. At least one tensioning member 568, such as but not limited to a tensioning cord, extends from proximal portion 562 to distal portion 564 and is coupled with the distal portion 564 and at least one tensioning actuator 570/572 on the proximal portion. Tensioning actuator 570/572 may include, for example, a knob 570 and a barrel 572 for wrapping and unwrapping tensioning member 568 to apply and remove tension. Tensioning member 568 is coupled with distal portion 564 at one or more connection points 580. In some embodiments, catheter device 560 includes a proximal housing 571, handle or the like, coupled to the proximal end of proximal portion 562 via a hub 576 or other means. Housing 571 may be coupled with tensioning actuator 570/572 and may include one or more arms 574 for infusing fluid or for other functions. In the embodiment shown, arm 574 and housing 571 include a lumen 567 that is in fluid communication with a fluid lumen 566 of the catheter body. Fluid may be introduced through arm 574 to pass through fluid lumen 566 to provide, for example, for contrast material at the distal tip of catheter device 560 to enhance visualization of device 560 during a procedure. Any other suitable fluid(s) may be passed through lumens 567/566 for any other purpose. Another lumen 578 may be included in distal portion 564, through which tensioning member 568 passes before attaching at a distal location along distal portion 564.

Fig. 15B shows catheter device 560 in a deformed/bent configuration, after tension has been applied to distal portion 564 by applying tension to tensioning member 568, via knob 570 and barrel 572. The bend in distal portion 564 will allow it to conform more readily to a valve annulus, while catheter device 560 in its straight configuration will be more amenable to passage through vasculature of the patient. Tensioning member 568 may be manufactured from any suitable material or combination of materials, such as but not limited to Nitinol, polyester, nylon, polypropylene and/or other polymers. Some embodiments may include two or more Tensioning members 568 and/or two or more tensioning actuators 570/572 to provide for changes in shape of distal portion 564 in multiple directions. In alternative embodiments, knob 570 and barrel 572 may be substituted with any suitable devices, such as a pull cord, button, lever or other actuator. Various alternatives may also be substituted for tensioning member 568 in various embodiments. For example, shaped expandable members, shape memory members and/or the like may be used to change the shape of distal portion 564.

Generally, proximal portion 562 of the catheter body is less flexible than distal portion 564. Proximal portion 562 may be made of any suitable material, such as PEBAX, FEP, nylon, polyethylene and/or the like, and may include a braided material, such as stainless steel, to provide stiffness and strength. Distal portion 564 may be made of similar or other materials, but the braided material is typically not included, to provide for greater flexibility. Both proximal and distal portions 562/564 may have any suitable lengths, diameters, overall configurations and the like. In one embodiment the catheter body is approximately 140 cm in length and 6 French in diameter, but any other suitable sizes may be used in other embodiments. Either proximal portion 562, distal portion 564 or preferably both, may be made from or coated with one or more friction resistant or lubricating material to enhance passage of device 560 through an introducer catheter and/or to enhance passage of a sheath or other device over catheter device 560.

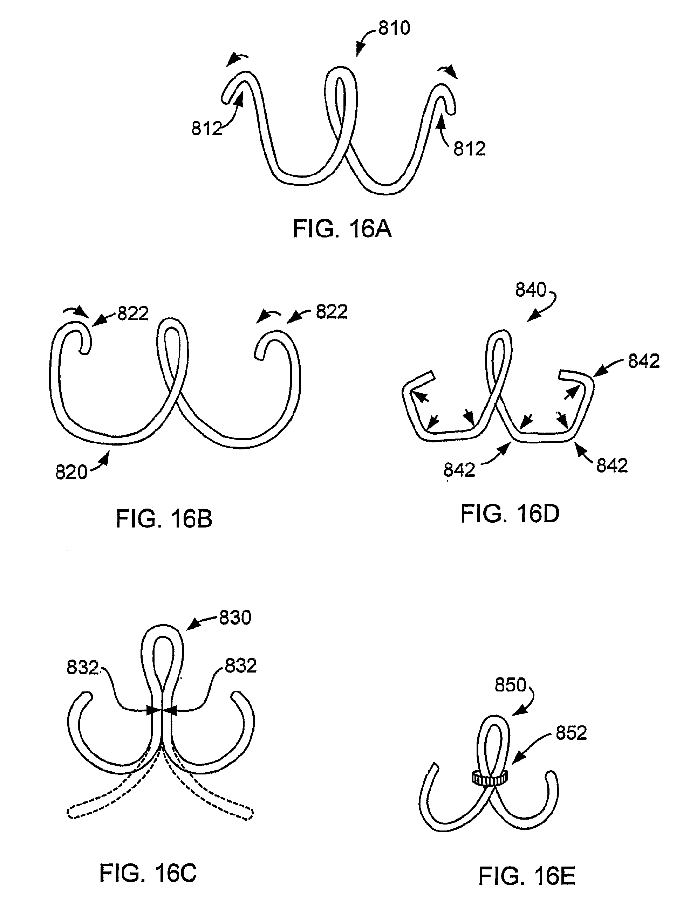

With reference now to Figs. 16A-16E, another aspect of the present invention includes improved tissue anchors for enhancing anchor attachment to valve annulus tissue. Such improved anchors typically include one or more features to help prevent the anchors from pulling out of tissue, when the anchors are placed under tension from a cinched tether, and/or to help promote tissue ingrowth of the anchors to further enhance attachment. In one embodiment, as shown in Fig. 16A, a tissue anchor 810 includes outwardly facing hooks 812 or bends at the ends of the two arms of anchor 810. In another embodiment, as in Fig. 16B, a tissue anchor 820 includes inwardly facing hooks 822. In a related embodiment, shown in Fig. 16D, a tissue anchor 840 includes multiple bends 842. In any of these embodiments, hooks 812, 822 or bends 842 have been found to enhance attachment of anchors 810, 820, 840 to tissue and thus prevent anchor pullout. In another embodiment, shown in Fig. 16C, two arms of a tissue anchor 830 are attached at an attachment point 832. The attachment point 832 may be formed by any suitable technique, such as soldering or the like. In another embodiment, as in Fig. 16E, a belt 852 may be disposed over a tissue anchor 850 to hold the two anns of the anchor together. In either of the embodiments shown in Figs. 16C and 16E, holding the two arms of the anchor together has be found to reduce pullout of the anchors 830, 850 from tissue.

In the embodiments just described or in alternative embodiments, tissue anchors may also have one or more features designed to enhance ingrowth and/or encapsulation of the anchors into annular tissue. Such features, for example, may include a coating, a porous and/or rough surface, an attachment such as a polyester band or belt, or any other suitable surface feature or added feature. By promoting encapsulation of tissue anchors, attachment strength of the anchors to tissue is enhanced.

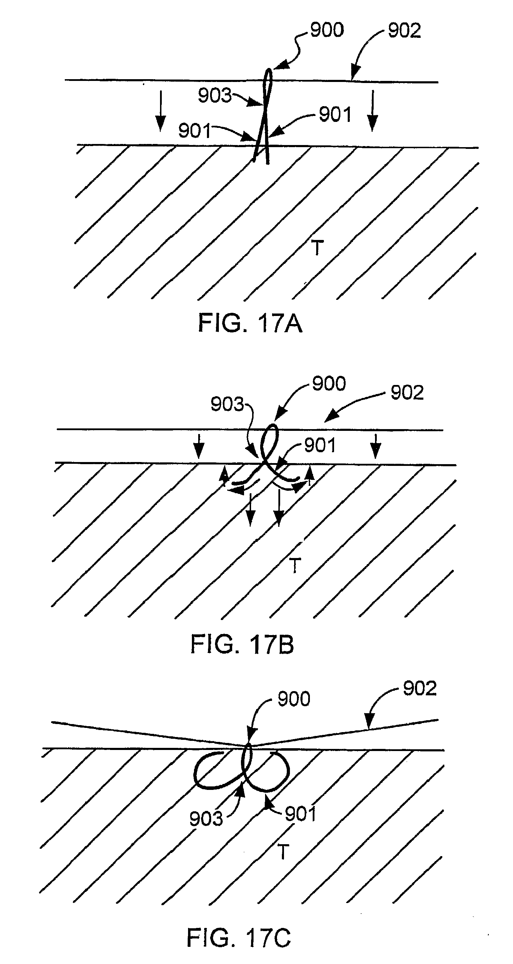

Referring now to Figs. 17A-17C, in many embodiments, self-forming anchors 900 are stored in the delivery device in a straightened configuration, coupled with a tether 902, as shown in Fig. 17A. Basically, anchors 900 are held or restrained in that straightened state, while their natural configuration is curved. Thus, when the straightened anchor 900 is released from the delivery device into tissue T, the anchor 900 actually pulls itself into the tissue T, as shown in Fig. 17B, due to the storage of potential energy in the straightened state and the tendency of each of the arms 901 of anchors 900 to drive the tip of the arm into the tissue as illustrated. Arms 901 are joined together at a junction 903. Each arm 901 is braced against the other arm so that forces exerted by tissue T on each arm 901 are opposed by the other arm 901 wherein the arms are joined to one another. This eliminates the need for an anchor driving device, such as required with staples, thus substantially simplifying the assembly and method. In addition, bracing arms 901 against one another also helps to reduce or eliminate problems associated with tissue deflection. As shown by the hollow-tipped arrows in Fig. 17B, the anchor 900 pulls itself into tissue T as it assumes its natural, curved shape, and exerts forces in vertical, horizontal and curved directions. Finally, after pulling itself into tissue and assuming its natural shape, as in Fig. 17C, anchor 900 is fully embedded in the tissue T.

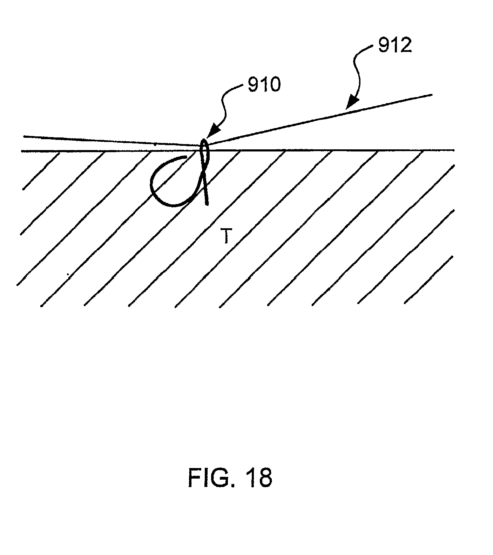

In an alternative embodiment, as shown in Fig. 18, anchors 910 may have one curved arm and one straight arm. Such an anchor 910 will still pull itself into tissue T, thus embedding itself and positioning the tether 912 flush with the tissue T.

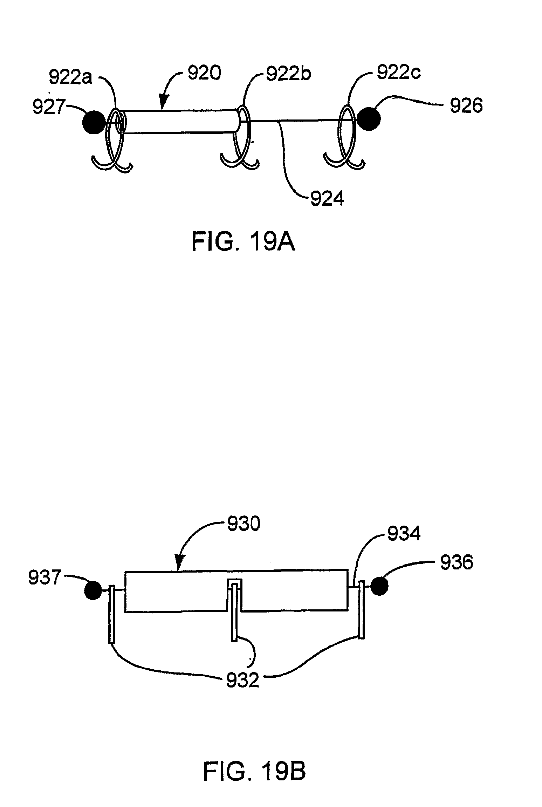

Referring now to Fig. 19A, some embodiments of a valve annulus anchor device may include anchors 922, a tether 924, a distal force applying member 927 coupled with the tether 924, a termination member 926 and one or more force distributing sleeves 920 disposed over the tether 924 and between adjacent anchors 922. In one embodiment, as shown, a separate sleeve 920 may be disposed between two adjacent anchors 922a, 922b. Additional sleeves 920 may optionally be disposed between other sets of two anchors, such as anchors 922b and 922c. In Fig. 19A, only three anchors 922 are shown for simplicity, but any number of anchors 922 and sleeves 920 between anchors may be used in various embodiments. Sleeve 920 acts to distribute force applied between two adjacent anchors 922, to help prevent such anchors 922 from pulling out of tissue when force is applied to tether 924. Sleeve 922 may be made of any suitable material, such as but not limited to metals, such as Nitinol, polymers, fabrics and the like. Sleeve 922 may be a solid cylindrical member, or alternatively may have patterned cut-outs, like a stent, or be made of ribbed, woven, braided, porous, nonporous or any other suitable material, pattern, configuration or the like. Sleeve 920 may be essentially rigid and axially incompressible, while in other embodiments it may be axially compressible. In one embodiment, sleeve 920 may be configured as two rings, disposed adjacent two anchors 922, with the rings being connected by a rod or shaft, so that tether 924 is not encircled by the sleeve 922.

With reference now to Fig. 19B, in an alternative embodiment, a sleeve 930 may be disposed over a tether 934 so as to extend between more than two anchors 932. Such a sleeve 930 may thus distribute force applied between a termination member 936 and a force applying member 937 so as to help prevent anchor pull-out from tissue. Such a sleeve 930 may include one or more openings through which one or more middle anchors may extend. Again, sleeve 930 may have any suitable configuration, size, shape or the like and be made of any suitable material or combination of materials. Sleeve 930 may extend between three, four, five or any suitable number of anchors 932 in various embodiments. In an alternative embodiment, sleeve 930 may be pierced by one or more of the anchors 932 and thus attached to valve annulus tissue.

Although the foregoing is a complete and accurate description of the present invention, the description provided above is for exemplary purposes only, and variations may be made to the embodiments described without departing from the scope of the invention. Thus, the above description should not be construed to limit the scope of the invention as described in the appended claims.
The claims of the parent application are reproduced below on pages 48-59. These clauses define preferred embodiments. The applicant reserves the right to pursue protection for the combinations of features set out in these clauses, and/or for any other subject-matter contained in the parent application as filed, either in the present divisional application or in a further application divided from the present divisional application. The claims of the parent application are not the claims of this divisional application. The claims of the current divisional application are contained in a separate section on pages numbered 60-61 and headed "Claims".

### Preferred Embodiments

1. A device for applying coupled anchors to an annulus of a heart valve, the device comprising: an elongate shaft having a proximal end and a distal end; a housing adjacent the distal end; a plurality of coupled anchors disposed within the housing; at least one anchor contacting member for causing the anchors to be delivered from the housing; and at least one actuator at or near the proximal end of the shaft for affecting the anchor contacting member to cause delivery of the anchors to the valve annulus.
2. A device as in clause 1, wherein the elongate shaft comprises a flexible catheter which may be advanced through an aorta and into a left ventricle of the heart to contact the valve annulus.
3. A device as in clause 2, wherein the housing has a cross-sectional diameter of about 1. 67 mm or less and a radius of each anchor, when deployed from the housing, is at least about 3 mm.
4. A device as in clause 1, wherein the housing is sufficiently flexible to be conformable to the annulus.
5. A device as in clause 4, wherein the housing conforms to the annulus at an intersection of a left ventricular wall and one or more mitral valve leaflets of the heart.
6. A device as in clause 5, further comprising at least one expandable member coupled with the housing for expanding to urge the housing toward the intersection to enhance contact of the housing with the valve annulus.
7. A device as in clause 5, wherein the housing has an oval or elliptical shape for helping to orient an anchor delivery surface of the housing to contact the valve annulus.
8. A device as in clause 4, wherein the housing is coupled with an actuator for deforming the housing to conform it to the annulus.
9. A device as in clause 4, wherein the housing is coupled with a shaped expandable member which expands to deform the housing to conform it to the annulus.
10. A device as in clause 4, wherein the housing comprises a shape-changing portion.
11. A device as in clause 10, further comprising at least a first tensioning cord coupled with the shape-changing portion for applying tension to the shape-changing portion to cause it to bend in at least a first direction.
12. A device as in clause 11, further comprising at least a second tensioning cord coupled with the shape-changing portion for applying tension to the shape-changing portion to cause it to bend in at least a second direction.
13. A device as in clause 12, wherein the first direction comprises approximately a C-shape for conforming to the annulus and the second direction comprises an upward or proximal direction for applying force to the annulus.
14. A device as in clause 11, wherein the shape-changing portion includes multiple notches along at least one side to control bending into a curve which conforms to the shape of the annulus.
15. A device as in clause 11, wherein the shape-changing portion comprises multiple stacked segments coupled with at least the first tensioning member to control bending into the shape of the annulus.
16. A device as in clause 11, wherein the shape-changing portion comprises a shape-memory material configured to conform to the shape of the annulus.
17. A device as in clause 16, wherein the shape-changing portion further comprises at least one lumen for introducing a fluid to cause the shape-memory material to conform to the shape of the annulus.
18. A device as in clause 1, wherein the housing comprises a plurality of openings, each opening sized to allow one of the plurality of anchors to be released.
19. A device as in clause 1, wherein each anchor has an undeployed shape when constrained in the housing by the at least one retaining member and elastically springs to a deployed shape when released from the housing.
20. A device as in clause 19, wherein the undeployed shape of each anchor is relatively straight, with each anchor having two sharpened tips, and wherein each anchor curves upon deployment, with the two tips curving in opposite directions.
21. A device as in clause 20, wherein the deployed shape of each anchor comprises two intersecting shapes, with an eyelet for passage of a slidable tether disposed at the intersection of the shapes.
22. A device as in clause 21, wherein the intersecting shapes are selected from the group consisting of circles, semicircles, helices, overlapping helices and ovals.
23. A device as in clause 19, wherein each anchor has an opened arcuate undeployed shape and assumes a closed shape with overlapping ends after release from constraint.
24. A device as in clause 23, wherein the undeployed shape is approximately a C- shape or semicircle having two sharpened ends, and the deployed shape is a closed circle in which the two ends overlap, wherein upon release from the delivery device the anchors secure to the annulus by penetrating the annulus with the ends and subsequently assuming the closed circle shape.
25. A device as in clause 1, wherein the anchors are slidably coupled with a tether.
26. A device as in clause 25, wherein each of the plurality of anchors includes at least one eyelet, and the tether passes through the at least one eyelet of each anchor.
27. A device as in clause 25, wherein the tether is disposed along the anchors so as to be positioned between the anchors and the valve annulus when the anchors are deployed.
28. A device as in clause 25, wherein the anchors are further coupled with a selfdeforming coupling member fixedly coupled with each anchor, the self-deforming member changing from an undeployed shape to a deployed shape upon release from the housing to cinch the anchors to tighten the annulus.
29. A device as in clause 25, further comprising a termination device slidably advanced over the tether, the termination device comprising: at least one attachment member for attaching the tether to at least a terminal anchor of the plurality of anchors; and at least one cutting member for cutting the tether to leave the cinched, tethered anchors secured to the valve annulus.
30. A device as in clause 1, further comprising at least one crimping member for crimping the coupled anchors to secure them to the annulus.
31. A device as in clause 1, wherein the anchors are coupled with a self-deforming coupling member fixedly coupled with each anchor, the self-deforming member changing from an undeployed shape to a deployed shape upon release from the housing to cinch the anchors to tighten the annulus.
32. A device as in clause 1, wherein the at least one anchor contacting member comprises at least one retractable force applying device which, when retracted proximally relative to the housing, sequentially contacts the anchors to apply force to the anchors such that they exit the housing via at least one opening in the housing.
33. A device as in clause 1, wherein the at least one anchor contacting member comprises at least one retaining member for retaining the anchors within the housing, wherein the at least one actuator moves the retaining member to release the anchors from the housing.
34. A device as in clause 1, wherein the at least one actuator includes means for cinching the coupled anchors to reduce the circumference of the valve annulus.
35. A device as in clause 1, further comprising at least one expandable member disposed within the housing for pushing the anchors out of the housing.
36. A device as in clause 1, further comprising at least one fastener coupled with the tether for securing the tether to at least one of the anchors after cinching the tether.
37. A device for applying multiple tethered anchors to an annulus of a heart valve, the device comprising: a flexible elongate catheter having a distal portion for delivering the tethered anchors, the distal portion having a cross-sectional diameter of about 1.67 mm or less; a plurality of tethered anchors disposed within the distal portion, each anchor having a radius of at least about 3 mm when deployed from the housing; and at least one anchor delivery member coupled with the catheter for causing the anchors to be delivered from the catheter.
38. A self-securing anchor for attaching to annular tissue of a heart valve, the selfsecuring anchor comprising a super-elastic or shape-memory material having a relatively elongate undeployed shape allowing the anchor to be disposed within a delivery catheter having a cross-sectional diameter of 1.67 mm or less, and assuming a deployed shape with a radius of at least 3 mm upon its release from the delivery device.
39. An anchor as in clause 38, wherein two sharpened tips of the anchor curve in opposite directions when the anchor is released from the delivery device.
40. An anchor as in clause 39, wherein the anchor comprises an eyelet disposed between the two sharpened tips.
41. A self-securing anchor for attaching to annular tissue of a heart valve, the selfsecuring anchor comprising a super-elastic or shape-memory material having an opened arcuate undeployed shape and assuming a closed shape with overlapping ends after release from constraint.
42. A device as in clause 41, wherein the undeployed shape is approximately a Cshape or semicircle having two sharpened ends, and the deployed shape is a closed circle in which the two ends overlap.
43. An anchor as in clause 41, wherein the anchor lies flush with the annular tissue when secured to the tissue.
44. An anchor as in clause 41, wherein the shape-memory material comprises Nitinol.
45. An anchor assembly for use within a patient comprising: a catheter-type housing, having an open interior, constructed to be passable through a blood vessel and into a heart; a series of tissue-engageable anchors removably housed within the housing, the anchors comprising a distal anchor and a proximal anchor within the open interior of the housing; and a tether serially coupling the anchors to one another.
46. The assembly according to clause 45 wherein at least a portion of the housing is flexible, and further comprising means for urging the flexible portion of the housing from a first configuration to a second, radially outwardly expanded, curved configuration.
47. The assembly according to clause 45 wherein the distal anchor is fixed to the tether and the proximal anchor is slidably coupled to the tether.
48. The assembly according to clause 45 wherein the tether is located substantially external of the catheter-type housing.
49. The assembly according to clause 45 wherein the anchors are self-forming and self-securing anchors.
50. The assembly according to clause 45 wherein at least one of the anchors comprises at least one of an electrode and a bioactive agent.
51. An anchor device for use within a patient comprising: an elongate housing having a longitudinal axis; a self-forming tissue-engageable anchor carried by and deployable from of the housing; the anchor having a first part and a second part, the second part having a tissue- piercing tip; the anchor placeable in a relatively straight, undeployed state generally parallel to the longitudinal axis when carried by the housing; and the anchor naturally assuming a curved, tissue-engaging deployed state after being deployed from the housing.
52. The device according to clause 51 wherein: the elongate housing has an open interior; the anchor is carried within and deployable from the open interior; and the housing has an opening sized for passage of the anchor therethrough when the anchor is deployed from the housing.
53. The device according to clause 51 wherein the anchor comprises said first part and at least two of said second parts extending from the first part.
54. The device according to clause 53 wherein the second parts extend in directions generally opposite one another when in the deployed state.
55. The device according to clause 51 wherein the anchor is oriented generally perpendicular to the longitudinal axis when in the deployed state.
56. The device according to clause 51 wherein the second part of the anchor has a generally circular or semicircular shape when in the deployed state.
57. The device according to clause 51 further comprising: a series of the anchors, the anchors comprising a distal anchor and a proximal anchor; and a tether serially coupling the anchors to one another.
58. The device according to clause 51 wherein the housing has the diametrical dimension d and the anchor in the deployed state has a diametrical dimension D, and wherein the ratio of D to d is at least 3.5.
59. The device according to clause 51 wherein the housing has the diametrical dimension d and the anchor in the deployed state has a diametrical dimension D, and wherein the ratio of D to d is at least 4.4.
60. The device according to clause 51 wherein the housing has the diametrical dimension d and the anchor in the deployed state has a diametrical dimension D, and wherein the ratio of D to d is at least 7.
61. The device according to clause 51 wherein the housing has the diametrical dimension d and the anchor in the deployed state has a diametrical dimension D, and wherein the ratio of D to d is at least 8.8.
62. A tissue anchor assembly comprising: an anchor comprising a base and a resilient, self-forming, tissue-engaging portion extending from the base, the tissue-engaging portion comprising a tissue-piercing tip; the tissue-engaging portion movable between a curved, unrestrained state and a restrained state; a coupling element coupled to the anchor; a housing; and the anchor releasably mounted to the housing with the tissue-engaging portion in the restrained state, the tissue-engaging portion transferable from the restrained state to the unrestrained state when the anchor is released from the housing so that the tissue-piercing tip passing into tissue secures the anchor to the tissue as the tissue-engaging portion moves to the curved, unrestrained state without any further force applied to the anchor.
63. The assembly according to clause 62 wherein the tissue-engaging portion is straightened when in the restrained state.
64. The assembly according to clause 62 wherein the anchor comprises first and second of the tissue-engaging portions, each of the first and second tissue-engaging portions comprises a hook which extend in directions generally opposite one another when in the unrestrained state.
65. The assembly according to clause 62 further comprising a plurality of said anchors with the coupling element coupled to each said anchor.
66. The assembly according to clause 65 wherein at least one said anchor is slidably coupled to the coupling element and another of said anchors is fastened to the coupling element.
67. A tissue anchor assembly comprising: a plurality of anchors, each anchor comprising a base and first and second self- forming, tissue-engaging portions extending from the base, the tissue-engaging portions each comprising a tissue-piercing tip; the tissue-engaging portions each movable between a curved, unrestrained state and a straightened, restrained state; the tissue-engaging portions each having a generally circular or semicircular shape when in the unrestrained state; a tether coupled to the bases of the anchors; at least one said anchor being slidably coupled to the tether and another of said anchors being fastened to the tether; a housing; and the anchors and tether being releasably mounted to the housing with the tissue- engaging portions in the restrained state, the tissue-engaging portions transferable from the restrained state to the unrestrained state when the anchors and tether are released from the housing so that the tissue-piercing tips passing into tissue secures the anchor to the tissue as the tissue-engaging portion moves to the curved, unrestrained state without any further force applied to the anchor.
68. A tissue anchor assembly comprising: an anchor comprising a base and a resilient, self-forming, tissue-engaging portion extending from the base, the tissue-engaging portion comprising a tissue-piercing tip; the tissue-engaging portion movable between a curved, unrestrained state and a restrained state; a coupling element coupled to the anchor; a housing; the anchor releasably mounted to the housing with the tissue-engaging portion in the restrained state, the tissue-engaging portion transformable from the restrained state to the unrestrained state when the anchor is released from the housing; and the tissue-engaging portion comprising means for pulling the anchor into the tissue as the tissue-piercing tip passes into tissue and the tissue-engaging portion moves to the curved, unrestrained state.
69. A tissue anchor assembly comprising: a plurality of anchors, each anchor comprising a base and a first and second resilient, self-forming, tissue-engaging portions extending from the base, the tissue-engaging portions each comprising a tissue-piercing tip; the tissue-engaging portions each movable between a curved, unrestrained state and a restrained state; the tissue-engaging portions each having a generally circular or semicircular shape when in the unrestrained state; a coupling element coupled to each of the anchors; a tubular housing; the anchors releasably mounted within the housing with the tissue-engaging portions in the restrained state, the tissue-engaging portions transformable from the restrained state to the unrestrained state when the anchors are released from the housing; the tissue-engaging portions comprising means for pulling the anchors into the tissue as the tissue-piercing tips pass into tissue and the tissue-engaging portions move to the curved, unrestrained states; and the anchor pulling means comprising means for storing potential energy when in the restrained state.
70. An anchor device for use within a patient comprising: an elongate carrier constructed to be passable through a blood vessel and into a heart; a series of tissue-engageable anchors releasably carried by the elongate carrier, the anchors comprising a distal anchor, a first intermediate anchor and a proximal anchor; a tether serially coupling the anchors to one another, the tether having a distal end secured to the distal anchor or engageable with the distal anchor when the tether is pulled in a proximal direction; at least the proximal anchor being slidably coupled to the tether; and a separator positioned along the tether between adjacent ones of the anchors to restrict how close the adjacent ones of the anchors can come to one another when the tether is placed in tension.
71. The device according to clause 70 wherein the anchors are self-forming and self-securing anchors.
72. The device according to clause 70 wherein the separator comprises a generally tubular member and wherein the tether passes through the generally tubular member.
73. The device according to clause 70 wherein the separator is axially compressible from a first length to a second, compressed length.
74. The device according to clause 70 wherein: the elongate carrier has a longitudinal axis; the anchors are self-forming tissue-engageable anchors; the anchors each have a first part and a second part, the second part having a tissue- piercing tip; the elongate carrier having openings sized for passage of the anchors therethrough; the anchors placeable in a relatively straight, undeployed state generally parallel to the longitudinal axis within the elongate carrier; and the anchors naturally assuming a curved, tissue-engaging deployed state after passing through the opening in the elongate carrier.
75. An anchor device for use within a patient comprising: an elongate carrier, having a longitudinal axis, constructed to be passable through a blood vessel and into a heart, at least a portion of the elongate carrier being flexible; a series of self-forming, tissue-engageable anchors releasably carried by the elongate carrier, the anchors comprising a distal anchor, a first intermediate anchor and a proximal anchor; the anchors each having a first part and at least two second parts extending from the first part, each second part having a tissue-piercing tip; the elongate carrier having openings sized for passage of the anchors therethrough; the anchors placeable in a relatively straight, undeployed state generally parallel to the longitudinal axis within the elongate carrier; the anchors naturally assuming a curved, tissue-engaging deployed state after passing through the opening in the elongate carrier; the second parts having generally circular or semicircular shapes when in the deployed state; the anchors being oriented generally perpendicular to the longitudinal axis when in the deployed state; the elongate carrier having a diametrical dimension d and the anchors in the deployed state having a diametrical dimension D, the ratio of D to d being at least 3.5; a tether serially coupling the anchors to one another, the tether having a distal end secured to the distal anchor or engageable with the distal anchor when the tether is pulled in a proximal direction; at least the proximal anchor being slidably coupled to the tether; and a separator positioned along the tether between adjacent ones of the anchors to restrict how close the adjacent ones of the anchors can come to one another when the tether is placed in tension.
76. A device for facilitating placement of one or more devices in contact with a heart valve annulus, the device comprising: an elongate catheter body having a proximal portion and a distal portion; at least one tensioning member coupled with the proximal portion of the catheter body and extending to the distal portion; and at least one tensioning actuator coupled with the proximal portion and the tensioning member for applying tension to the tensioning member to deform the distal portion to allow it to conform generally to a shape of the valve annulus.
77. A device as in clause 76, wherein the at least one tensioning member comprises two tensioning members, allowing the distal portion to be deformed in at least two different directions.
78. A device as in clause 76, further comprising an urging member coupled with the distal portion of the catheter body for urging the distal portion into contact with the valve annulus.
79. A device as in clause 78, wherein the urging member comprises at least one of (a) an expandable member for expanding within a space in a left ventricle formed by a left ventricular wall, at least one mitral valve leaflet and chordae tendiniae of the heart, and (b) at least one magnet coupled with the distal portion for applying attractive magnetic force between itself and an oppositely charged magnet disposed in a coronary sinus adjacent the valve annulus.
80. A system for facilitating placement of one or more devices in contact with a heart valve annulus, the system comprising: a shaped guide catheter having at least one curve toward a distal end for positioning the distal end in a position below the mitral valve; a steerable guide catheter passable through the shaped guide catheter and having a steerable distal end for advancing around a length of the valve annulus below the mitral valve; and a guide sheath passable over the steerable guide catheter through the shaped guide catheter, wherein the one or more devices are passable through the guide sheath to contact the mitral valve annulus.
81. A system as in clause 80, wherein the at least one curve of the shaped guide catheter comprises: a proximal curve approximately perpendicular to a central axis of the shaped guide catheter for bringing the distal end of the catheter into a plane approximately parallel with a plane of the mitral valve; and a distal curve having a radius of curvature approximately the same as a radius of curvature of the mitral valve annulus.
82. A system as in clause 80, wherein a distal portion of the guide sheath is detachable from a proximal portion of the guide sheath to remain in attached to the valve annulus after an annulus treatment procedure.
83. A system as in clause 82, wherein the detachable distal portion is cinchable to tighten the mitral valve annulus.
84. A system as in clause 80, further comprising an anchor delivery device passable through the guide sheath to contact and apply coupled anchors to the mitral valve annulus.
85. A system as in clause 80, further comprising a visualization device passable through the guide sheath to facilitate visualization of the mitral valve annulus.
86. A device for performing a procedure on heart valve annulus, the device comprising: a flexible, elongate catheter having a proximal portion and a shaped distal portion, the distal portion having at least one aperture for allowing passage of tissue anchors; multiple tissue anchors releasably housed in the shaped distal portion; at least one cinchable tether slidably coupled with the tissue anchors; and at least one anchor delivery member housed in the distal portion for delivering the anchors out of the at least one aperture to engage tissue of the valve annulus.
87. A device as in clause 86, wherein the shaped distal portion comprises a first curve with a radius of curvature between 1.3 mm and 3.8 mm.
88. A device as in clause 87, wherein the shaped distal portion further comprises a second curve.
89. A device as in clause 88, wherein the first and second curves orient a distal- most portion of the catheter at an angle of between 45 and 90, relative to the proximal portion of the catheter immediately adjacent the distal portion.
90. A device as in clause 88, wherein the second curve has a radius of curvature greater than a radius of curvature of the heart valve, wherein the greater radius of curvature causes the distal portion to press outward against the valve annulus.
91. A device as in clause 88, wherein at least one of the first and second curves has the same shape as a corresponding curve in a guide catheter used for delivering the elongate catheter into contact with the valve annulus.
92. A device as in clause 91, wherein at the first and second curves in the elongate catheter correspond to first and second corresponding curves in the guide catheter.
93. A device as in clause 91 wherein the elongate catheter, when advanced through the guide catheter, is oriented such that the at least one aperture contacts the valve annulus tissue.
94. A device as in clause 86, further comprising at least one stabilizing member coupled with the elongate catheter for maintaining the distal portion in contact with valve annulus tissue.
95. A device as in clause 94, wherein the at least one stabilizing member comprises at least one of: a spiral-shaped member extending from the distal portion to press against heart wall tissue, thus urging the distal portion against the annulus tissue; an arch-shaped shape-memory or spring-loaded member extending from the distal portion to press against heart wall tissue, thus urging the distal portion against the annulus tissue; multiple springs extending from the distal portion to press against heart wall tissue, thus urging the distal portion against the annulus tissue; and a curved balloon coupled with the distal portion of the catheter, the curved balloon having a greater radius of curvature than a radius of curvature of the valve annulus, wherein inflating the balloon urges the distal portion against the annulus tissue.
96. A device as in clause 86, further comprising at least one termination member for enhancing attachment of a terminal tissue anchor to the heart valve annulus.
97. A device as in clause 96, wherein the at least one termination member is slidably couplable with the tether.
98. A device as in clause 86, wherein each of the tissue anchors comprises at least one support member for preventing the anchors from being pulled out of the valve annulus tissue when the tether is cinched.
99. A device as in clause 86, wherein each of the tissue anchors comprises at least one tissue adhesion feature adapted to enhance attachment of the tissue anchors to the valve annulus tissue.
100. A device as in clause 99, wherein the tissue adhesion feature comprises one or more materials disposed over at least part of each anchor, the material (s) selected to promote encapsulation of the anchors within the valve annulus tissue.
101. A device as in clause 86, wherein the at least one anchor delivery member comprises: an anchor contacting member for contacting and urging the anchors out of the at least one aperture; and a pull cord coupled with the anchor contacting member for applying force to the anchor contacting member to contact and urge the anchors.
102. A device for constricting a valve annulus in a heart, the device comprising: a plurality of slidably coupled tissue anchors, each anchor including at least one tissue attachment feature for enhancing attachment of the anchor to valve annulus tissue; and at least one cinchable tether slidably coupled with at least some of the tissue anchors and fixedly attached to at least a first of the anchors.
103. A device as in clause 102, wherein the at least one attachment feature comprises at least one of: a porous surface along at least a portion of each anchor; a textured surface along at least a portion of each anchor; a coated surface along at least a portion of each anchor; at least one circular or hooked portion of each anchor having a small radius of curvature; and a barb at each end of each anchor.
104. A catheter assembly comprising: a guide sheath having a guide sheath axis; an elongate device slidably extendable within the guide sheath, the elongate device having an elongate device axis; the guide sheath comprising a sheath orientation portion placeable in a chosen shape; and the elongate device comprising a device orientation portion placeable in said chosen shape; whereby the chosen shape of the sheath and device orientation portions tend to cause the sheath and device orientation portions to assume complementary rotary orientations when the sheath and device orientation portions are axially aligned.
105. The catheter assembly according to clause 104 wherein at least a chosen one of the guide sheath and elongate device are steerable.
106. The catheter assembly according to clause 104 wherein the sheath orientation portion is located at a distal end of the guide sheath.
107. The catheter assembly according to clause 104 wherein the device orientation portion is located at a position spaced apart from a distal end of the elongate device.
108. The catheter assembly according to clause 104 wherein said chosen shape comprises a first curved portion in a first plane and a second curved portion in a second plane, said first and second planes being different planes.
109. A method for creating a catheter assembly having orientation-seeking inner and outer members comprising: selecting a first chosen location for a sheath orientation portion of a sheath; selecting a second chosen location for an elongate device orientation portion of an elongate device, the elongate device slidably extendable within the guide sheath; creating the sheath orientation portion at the first chosen location of the sheath, the sheath orientation portion placeable in a chosen shape; and creating the elongate device orientation portion at the second chosen location of the sheath, the device orientation portion placeable in said chosen shape, whereby the chosen shape of the sheath and device orientation portions tend to cause the sheath and device orientation portions to assume complementary rotary orientations when the sheath and device orientation portions are axially aligned.
110. The method according to clause 109 wherein the first chosen location selecting step comprises selecting the distal end of the guide sheath as the first chosen location.
111. The method according to clause 109 wherein the second chosen location selecting step comprises selecting a position spaced apart from a distal end of the elongate device as the second chosen location.
112. The method according to clause 109 wherein the sheath orientation portion creating step comprises forming a first curved portion and a second curved portion.
113. A method for orienting the inner and outer members of a catheter assembly comprising: selecting a catheter assembly comprising: a guide sheath having a guide sheath axis and a distal end; an elongate device slidably extendable within the guide sheath, the elongate device having an elongate device axis; the guide sheath comprising a sheath orientation portion, the sheath orientation portion placeable in a chosen shape; and the elongate device comprising a device orientation portion, the device orientation portion placeable in said chosen shape; placing the distal end of the guide sheath at a chosen location; axially aligning the sheath and device orientation portions so that the guide sheath and elongate device are at a chosen axial orientation; and axially repositioning, as necessary, the guide sheath and elongate device away from and back into the chosen axial orientation until the sheath and device orientation portions to assume complementary rotary orientations.
114. A method for securing an anchor to tissue of a patient comprising: directing an anchor assembly, comprising an anchor carried by an anchor carrier, to a target tissue site; and releasing the anchor from the anchor carrier, the releasing step comprising: placing a tissue-piercing tip of the anchor into engagement with the tissue at the target tissue site; and the anchor pulling itself into the tissue as the anchor moves from a restrained state to an unrestrained state.
115. A method for advancing one or more devices into a left ventricle of a heart to contact a mitral valve annulus, the method comprising: advancing a steerable guide catheter into the left ventricle and around at least a portion of the mitral valve annulus; passing a guide sheath over the steerable guide catheter; withdrawing the steerable guide catheter out of the guide sheath; and advancing one or more devices through the guide sheath to contact the mitral valve annulus.
116. A method for advancing one or more devices into a left ventricle of a heart to contact a mitral valve annulus, the method comprising: advancing a shaped guide catheter through an aorta into the left ventricle; passing a steerable guide catheter through the shaped guide catheter and around at least a portion of the length of the mitral valve annulus; passing a guide sheath over the steerable guide catheter, within the shaped guide catheter; withdrawing the steerable guide catheter out of the guide sheath; and advancing one or more devices through the guide sheath to contact the mitral valve annulus.
117. A method for treating a mitral valve annulus of a heart, the method comprising: advancing a steerable guide catheter into a left ventricle of the heart and around at least a portion of the mitral valve annulus; passing a guide sheath over the steerable guide catheter; withdrawing the steerable guide catheter out of the guide sheath; advancing an anchor delivery device through the guide sheath to contact the mitral valve annulus; delivering a plurality of coupled anchors from the anchor delivery device to secure the anchors to the mitral valve annulus; and drawing the anchors together to circumferentially tighten the annulus.
118. A method of constricting a valve annulus in a heart, the method comprising: contacting a shaped distal portion of an anchor delivery catheter device with a length of the valve annulus; delivering a plurality of slidably coupled anchors from the anchor delivery device to secure the anchors to the annulus; drawing the anchors together to circumferentially tighten the valve annulus; and promoting attachment of the anchors to valve annulus tissue to help secure the anchors to the tissue over time.
119. A method for advancing an operational device into a left ventricle of a heart to contact the mitral valve annulus comprising: advancing an operational device into a left ventricle and along at least a portion of a mitral valve annulus of a heart; urging the operational device radially outwardly to seat the operational device against the mitral valve annulus; and acting on the mitral valve annulus by the operational device.
120. A method for advancing an operational device into a left ventricle of a heart to contact and circumferentially tighten the mitral valve annulus comprising: selecting an operational device comprising an elongate housing having a diametrical dimension d and anchors having a diametrical dimension D in a deployed state, and wherein the ratio of D to d is at least 3.5; advancing the operational device through an aorta and into a left ventricle and along at least a portion of a mitral valve annulus of a heart of a patient, the advancing step being carried out using an operational device comprising: an elongate housing having a longitudinal axis and an open interior; a series of self-forming tissue-engageable anchors, the anchors comprising a distal anchor and a proximal anchor within the open interior of the housing; the anchor having a first part and at least two second parts extending from the first part, the second parts each having a tissue-piercing tip, the second parts having generally circular or semicircular shapes when in the deployed state; a tether serially coupling the anchors to one another with the proximal anchor coupled to the tether and the distal anchor secured to the tether; the housing having openings sized for passage of the anchors tip-first through the opening; and each of the anchors placeable in a relatively straight, undeployed state generally parallel to the longitudinal axis within the housing; urging the operational device radially outwardly to seat the operational device against the mitral valve annulus; securing the series of anchors to the mitral valve annulus by: driving the anchors tip-first through the openings with the anchors naturally assuming curved, tissue-engaging deployed states after passing through the openings in the housing, the anchors oriented generally perpendicular to the longitudinal axis when in the deployed state; and pulling on the tether to reduce the circumferential distance between the proximal and distal anchors and circumferentially tighten the mitral valve annulus.
121. A method for advancing an operational device into a left ventricle of a heart to contact the mitral valve annulus comprising: advancing a guide catheter into a left ventricle and along at least a portion of a mitral valve annulus of a heart; passing a flexible guide sheath over the guide catheter and along at least a portion of the mitral valve annulus; advancing an operational device through the guide sheath; urging the operational device radially outwardly to seat the operational device against the mitral valve annulus; and acting on the mitral valve annulus by the operational device.
122. A method for advancing an operational device into a left ventricle of a heart to contact the mitral valve annulus comprising: advancing an operational device into a left ventricle and along at least a portion of a mitral valve annulus of a heart; urging the operational device radially outwardly to seat the operational device against the mitral valve annulus; the urging step being carried out at least in part by virtue of the operational device having a radius of curvature when in an expanded state larger than the radius of curvature of the mitral valve annulus; and acting on the mitral valve annulus by the operational device.
123. A method for advancing an operational device into a left ventricle of a heart to contact the mitral valve annulus comprising: advancing an operational device into a left ventricle and along at least a portion of a mitral valve annulus of a heart, the operational device comprising an expansible element; the operational device advancing step comprising steering the operational device; radially outwardly expanding the expansible element to urge the operational device radially outwardly to seat the operational device against the mitral valve annulus; and acting on the mitral valve annulus by the operational device.
124. A method for advancing an operational device into a left ventricle of a heart to contact the mitral valve annulus comprising: advancing a guide catheter into a left ventricle and along at least a portion of a mitral valve annulus of a heart, the mitral valve annulus having a mitral valve annulus radius; selecting a flexible guide sheath having a curveable portion, said curveable portion having a guide sheath radius when in an outwardly expanded curved state, the guide sheath radius being larger than the mitral valve annulus radius; passing the guide sheath over the guide catheter and along at least a portion of the mitral valve annulus; urging the curveable portion of the guide sheath in the curved state towards the mitral valve annulus at least in part by virtue of the guide sheath radius; advancing an operational device through the guide sheath; and acting on the mitral valve annulus by the operational device.
125. A method for advancing an operational device into a left ventricle of a heart to contact the mitral valve annulus comprising: selecting a flexible guide catheter having a curveable portion, said curveable portion having a guide catheter radius when in outwardly expanded curved state; advancing the guide catheter into a left ventricle and along at least a portion of a mitral valve annulus of a heart, the mitral valve annulus having a mitral valve annulus radius; the selecting step being carried out with the guide catheter radius being larger than the mitral valve annulus radius; passing a curveable portion of the a guide sheath over the guide catheter and along at least a portion of the mitral valve annulus; urging the curveable portion of the guide sheath towards the mitral valve annulus at least in part by virtue of the guide catheter radius; advancing an operational device through the guide sheath; and acting on the mitral valve annulus by the operational device.
126. A method of constricting a valve annulus in a heart, the method comprising: contacting an anchor delivery device with a length of the valve annulus; delivering a plurality of coupled anchors from the anchor delivery device to secure the anchors to the annulus; and drawing the anchors together to circumferentially tighten the valve annulus.
127. A method of constricting a valve annulus in a heart, the method comprising: contacting an anchor delivery device having a cross-sectional diameter of about 1.67 mm or less with a length of the valve annulus; delivering a plurality of coupled anchors from the anchor delivery device to secure the anchors to the annulus, each anchor having a deployed shape with a radius of at least about 3 mm; and drawing the anchors together to circumferentially tighten the valve annulus.

## Claims

1. A system for facilitating placement of one or more devices in contact with a length of tissue in a subannular space, the system comprising:
a guide sheath having a distal portion and a sheath orientation portion, the sheath orientation portion having a pre-shaped curved configuration, and wherein the guide sheath is configured to position the distal portion about a subannular space; and
an elongate device that is slidably extendable through the guide sheath to contact a length of tissue in the subannular space, wherein the elongate device comprises a distal end and a device orientation portion having a pre-shaped curved configuration complementary to the curved shape of the sheath orientation portion such that the sheath and device orientation portions may be aligned so that the sheath and device orientation portions may form a complementary interfit.

2. The system of claim 1, wherein the device orientation portion has a radius of curvature that is generally similar to or greater than a radius of curvature of the sheath orientation portion.

3. The system of claim 1, wherein the sheath and device orientation portions comprise orientation markers that are configured to be visually aligned.

4. The system of claim 1, wherein alignment of the sheath and device orientation portions is sensed tactilely.

5. The system of claim 1, wherein the elongate device is an anchor delivery device comprising an elongate catheter having a housing at or near a distal end of the catheter, and wherein the anchor delivery device is passable through the guide sheath to contact and apply an anchor to the length of tissue in the subannular space.

6. The system of claim 1, further comprising a visualization device passable through the guide sheath to facilitate visualization of the length of tissue in the subannular space, wherein the visualization device is selected from the group consisting of an ultrasound device, a camera, an endoscope, and a fiber optic device.

7. The system of claim 1, wherein the elongate device has a plurality of distinct anchor delivery sites.

8. The system of claim 1, wherein the sheath orientation portion is placeable in a chosen shape, and the device orientation portion is placeable in the chosen shape; whereby the chosen shape comprises one or more curved portions in a plurality of different planes.

9. The system of claim 5, wherein the housing of the elongate catheter comprises a shape-changing portion.

10. The system of claim 5, wherein the sheath orientation portion is placeable in a chosen shape, and the device orientation portion is placeable in the chosen shape;
whereby the chosen shape of the sheath and anchor delivery device orientation portions tend to cause the sheath and device orientation portions to assume complementary rotary orientations when the sheath and device orientation portions are axially aligned.

11. The system of claim 10 wherein the sheath orientation portion is located at a distal portion of the guide sheath.

12. The system of claim 10 wherein the device orientation portion is located at a position spaced apart from the distal end of the anchor delivery device catheter.

13. The system of claim 10 wherein the chosen shape comprises a first curved portion and a second curved portion.

14. The system of claim 10 wherein the chosen shape comprises a first curved portion in a first plane and a second curved portion in a second plane.

15. The system of claim 10 wherein the chosen shape comprises a first curved portion in a first plane and a second curved portion in a second plane, the first and second planes being different planes.
